# EUROPEAN PATENT APPLICATION

(11) **EP 2 527 359 A2**
(43) Date of publication of application: **28.11.2012**
(21) Application number: 12150847.7
(22) Date of filing: 03.04.2008
(51) Int. Cl.: C07K 7/08, G01N 33/80

(54) **Diagnostic assay**

(30) Priority: 03.04.2007 GB 0706558
(62) Divisional of application: 08736890.8
(71) Applicant: THE COMMON SERVICES AGENCY FOR THE SCOTTISH HEALTH SERVICE, Edinburgh Lothian EH12 9EB (GB)
(72) Inventor: Urbaniak, Stanislaw, Aberdeen, Aberdeenshire AB11 7SY (GB); Moss, Michael, Kemnay, Aberdeenshire AB51 5ST (GB); Tait, Evelyn, Aberdeen, Aberdeenshire AB25 1ST (GB)
(74) Representative: Williams, Paul Edwin

(57) **Abstract**

The present invention relates to mimotopes of blood group antigens, methods for identifying mimotopes of blood group antigens and methods for identifying antibodies to blood group antigens using said mimotopes.

## Description

The present invention relates to mimotopes of blood group antigens, and uses thereof.

Blood group antigens exist on membrane proteins on the surface of erythrocytes. The genes encoding all but one of the 29 blood group systems (Rhesus, Kell, Duffy, Kidd, ABO, MNS, P, Lutheran, Lewis, Diego, Yt, Xg, Scianna, Dombrock, Colton, Landsteiner-Wiener, Chido-Rodgers, Hh, Kx, Gerbich, Cromer, Knops, Indian, Ok, Raph, JMH, I, Globoside and GIL) have been cloned, sequenced, the amino acid sequences deduced, and the membrane structure predicted.

Of the Rhesus, Kell, Duffy and Kidd blood systems etc, the Rhesus antigens are the most complex being the products of two highly homologous genes, RHD and RHCE. The antigens are predicted to have six extracellular loops that are available for antibody binding.

The RhD polypeptide, the most commonly expressed of the Rhesus antigens, differs by 36 amino acids from its nearest equivalent, the RhCcEe polypeptide. Both are co-expressed with the Rhesus-associated glycoprotein (RAG) polypeptide.

The RHCE gene product exists in four allelic forms and each allele determines the expression of two antigens in combination i.e. Ce, ce, cE or CE. The RhC antigen differs from Rhc by one amino acid in loop 2 (Serl03Pro), and for E/e, a single exofacial amino acid difference is predicted in loop 4 (Pro226Ala).

The Kell protein is a single pass membrane structure, and the K/k alleles are the result of a single nucleotide polymorphism (SNP) resulting in a single amino acid change (Met193Thr) in the extracellular region.

The Duffy protein is predicted to have 3 extracellular loops with the N-terminus being extracellular, and the Fy^{a}/Fy^{b} alleles are the result of a SNP giving rise to an amino acid substitution (Asp42Gly) in the N-terminal region.

The Kidd glycoprotein is predicted to have 10 membrane-spanning domains with intracellular N- and C-termini. The Jk^{a}/Jk^{b} polymorphism results from a SNP giving rise to a single amino acid substitution (Asp280Asn) on the fourth extracellular loop.

The immunological determinants on an antigen that bind antibodies are defined as B-cell epitopes since they are recognised by the surface-membrane immunoglobulin receptors of B-lymphocytes. These epitopes may be linear i.e. a continuous stretch of amino acids from the protein sequence, or conformational and depend on the spatial juxtaposition of amino acids which are not contiguous. Study of the B-cell epitope structure of blood group antigens has largely been restricted to the RhD antigen, and to date over 30 epitopes have been identified serologically.

A single amino acid substitution can affect more than one epitope, even though the predicted loops involved are some distance apart in the linear sequence, and it is concluded that the RhD B-cell epitopes are conformational.

The B-cell epitopes on C/c and E/e are predicted to be fewer, but are likely to be conformational since full antigenicity requires co-expression of the RAG polypeptide.

The K/k polymorphism is predicted to disrupt a glycosylation site, which may account for the antigenicity of the K allele, and it is anticipated that the epitopic determinants will also be conformational.

Blood group antigens may induce the production of alloantibodies that can cause destruction of transfused red blood cells. Reactions to transfused blood may, therefore, occur when a recipient of transfused erythrocytes has antibodies to those erythrocytes, resulting in their destruction. An immediate reaction including fever, hypotension, nausea and vomiting, and pain in the back and chest can occur, with the severity of the reaction depending on the class and amount of antibodies involved.

Alloantibodies to blood group antigens may also cross the placenta of a pregnant woman and give rise to haemolytic disease of the newborn (HDNB). Haemolytic disease of the newborn (HDNB) can occur when the mother has been sensitised to antigens on the infant's erythrocytes and makes IgG antibodies to these antigens. These antibodies can cross the placenta and react with the foetal erythrocytes causing their destruction. RhD is the most commonly involved antigen.

Blood group antigens may also induce the production of autoantibodies involved in autoimmune diseases such as autoimmune haemolytic anaemia. The trigger for this autoimmune disease is unknown and therefore it may occur at anytime and results in the body producing autoantibodies of broad Rh group specificity which attack the body's own red blood cells.

The most important blood groups associated with alloantibody generation are Rhesus, Kell, Duffy, and Kidd.

Current methods for detecting alloantibodies that may destroy transfused erythrocytes or cross the placenta to cause HDNB, or for detecting autoantibodies, rely on blood donor red cell reagents. These have a short shelf-life and vary considerably in the antigen expression between donors. Use of human blood products also introduces safety implications, for example, involving the potential transfer of viral infections. The rigorous testing and screening required when using human reagents, together with inconsistencies between samples slows the availability of reagents for detecting alloantibodies, as well as increasing the cost of obtaining suitable reagents.

The present invention, accordingly, seeks to overcome the problems associated with the prior art, in particular, the problems associated with existing methods for detecting alloantibodies and autoantibodies.

According to an aspect of the present invention there is provided a mimotope of a blood group antigen.

The term "mimotope" refers to a peptide which is capable of mimicking an epitope of a native peptide. Usually such peptides are synthetic peptides. In this respect, "synthetic peptide" includes any peptides which are synthesised rather than expressed natively in a biological system. Synthesis may be by recombinant technology such as phage display, F-moc chemistry or any other method for expressing or synthesising foreign peptides in biological or non-biological systems or environments.

The present invention thus provides novel peptides which are capable of binding to antibodies which have been raised to blood group antigens. These peptides are not necessarily from natural sources such as human blood and, therefore, do not carry the health risks, storage problems and variability associated therewith.

Conveniently, the blood group antigen is selected from Rhesus, Kell, Duffy, Kidd, ABO, MNS, P, Lutheran, Lewis, Diego, Yt, Xg, Scianna, Dombrock, Colton, Landsteiner-Wiener, Chido-Rodgers, Hh, Kx, Gerbich, Cromer, Knops, Indian, Ok, Raph, JMH, I, Globoside or GIL.

The deduced amino acid structure of a mimotope may not necessarily correspond to the amino acid structure of the antigen which it mimics. Indeed, in the present study it was discovered that the majority of the blood group mimotopes identified have amino acid sequences which differ widely from the amino acid sequence of the native antigen that they mimic.

As the epitopes of many blood group antigens are thought to be complex in conformation, it is surprising that the synthetic peptides, identified in the present invention by means of immunopanning, are capable of binding antibodies raised to native antigens in a specific manner. This finding suggests that the synthetic blood group mimotopes assume and maintain a three dimensional conformation which allows them to specifically bind the antibodies raised to their corresponding antigen.

Conveniently, the mimotope is synthesised. This removes the problems associated with using human blood reagents, for example, the potential risk of contamination and infection, difficulties regarding storage, donor identification, variability and screening. These problems have associated costs which will be reduced through use of synthetic mimotopes. Use of synthetic mimotopes also provides a greater degree of control over the reagents that are available for use, ensuring that shortages never occur and unnecessary waiting for reagents can be avoided.

Through the present study, numerous mimotopes of blood group antigens were identified. Some of the peptides conveniently identified as blood group mimotopes are identified in Tables 2-5 and include peptide fragments, immunoreactive analogues or derivatives thereof.

According to a further aspect of the present invention there is provided a method for identifying a mimotope of a blood group antigen, the method comprising contacting a random phage display library with an antibody to a blood group antigen and detecting binding of the antibody to peptides in the phage display library, wherein binding of an antibody to a peptide is indicative of the peptide being a mimotope of a blood group antigen.

Preferably, the blood group antigen is Rhesus, Kell, Duffy, Kidd, ABO, MNS, P, Lutheran, Lewis, Diego, Yt, Xg, Scianna, Dombrock, Colton, Landsteiner-Wiener, Chido-Rodgers, Hh, Kx, Gerbich, Cromer, Knops, Indian, Ok, Raph, JMH, I, Globoside or GIL. These are the blood group antigens associated with the generation of alloantibodies and autoantibodies.

The antibody to which the mimotope binds may be a monoclonal antibody or a preparation of polyclonal antibodies. Methods for generating monoclonal antibodies and polyclonal antibodies are well known in the art.

Random phage display libraries comprising 7-mer, 12-mer and 15-mer peptides were used to identify peptides which could bind antibodies raised to native blood group antigens. Identification and sequencing of peptides which bound to the antibodies enabled identification of potential blood group antigen mimotopes. Subsequent analysis of these peptides by ELISA followed by synthesis of these peptides by SPOTs and analysis of the membrane-bound SPOTs confirmed the strength and specificity of the peptide-antibody binding. This confirmed that synthetic peptides structurally different from the native antigens which they mimicked were successfully able to specifically bind antibodies raised to native blood group antigens, thus representing effective mimotopes of the blood group antigens in question. Immobilisation of the mimotopes onto, for example, microsphere beads or superparamagnetic beads did not affect the ability of the mimotopes to specifically bind antibodies raised to native blood group antigens. The method described therefore represents an effective and efficient method for identifying synthetic mimotopes of blood group antigens which can be used in a clinical setting, for example, in the diagnosis of diseases associated with the production of alloantibodies or autoantibodies.

Binding of antibodies to the phage-peptides may be detected by any means suitable, such means being known to a person skilled in the art. Examples of the type of binding which can be used is described in the Examples.

According to a further aspect of the present invention there is provided a method for detecting an antibody to a blood group antigen in a sample. The method comprising reacting a mimotope of said blood group antigen with the sample to be tested, detecting any reaction between the sample and the mimotope, wherein a reaction between the sample and the mimotope is indicative of the presence of an antibody to the blood group antigen.

Conveniently before carrying out the above identified method the mimotope is immobilised on a solid support. Immobilisation of the mimotopes onto a solid support such as microsphere beads or superparamagnetic beads did not affect the ability of the mimotopes to specifically bind antibodies raised to native blood group antigens. In this connection, the method comprises immobilising a mimotope of a blood group antigen on a solid support, contacting the immobilised mimotope with the sample to be tested and detecting binding of the sample to the mimotope, wherein binding of the sample to the mimotope is indicative of the presence of an antibody to the blood group antigen mimicked by the mimotope.

Conveniently, the blood group antigen is Rhesus, Kell, Duffy, Kidd, ABO, MNS, P, Lutheran, Lewis, Diego, Yt, Xg, Scianna, Dombrock, Colton, Landsteiner-Wiener, Chido-Rodgers, Hh, Kx, Gerbich, Cromer, Knops, Indian, Ok, Raph, JMH, I, Globoside or GIL.

In this respect, given that many epitopes of native blood group antigens are thought to be conformational it is surprising that the synthetic mimotopes identified herein are capable of presenting a three dimensional conformation which specifically bind antibodies raised to native blood group antigens, even when bound to a solid support. The solid support may comprise any support suitable for the purpose but conveniently comprises a membrane, glass slide or bead (glass, polystyrene etc.). Attachment to the support can be by any suitable means, for example using Ni-NTA groups attached to the surface of polystyrene microsphere beads or using superparamagnetic polystyrene beads. Polyhistidine tags may be incorporated into the C-terminal or N-terminal of mimotopes to aid attachment to the support. Membranes having a lower background and which can be successfully stripped and re-probed, for example INTAVIS AG membranes, are preferable.

The present invention, therefore, provides a method which effectively identifies antibodies in biological samples that have been raised to blood group antigens. Use of synthetic mimotopes removes the problems identified with regard to use of human reagents. This will increase consistency in antibody detection and enable detection of rarer antibodies for little or no extra cost, compared with existing techniques for screening antibodies raised to blood group antigens. Avoiding use of human blood products also increases safety, avoids the rigorous testing required when using human blood products and increases the consistency of the reagents available for use. This reduces the cost of sourcing and clearing reagents for use. The method described is therefore more reliable, safe and convenient than existing detection methods, making detection of antibodies to blood group antigens in biological samples quicker, cheaper and easier to conduct.

The antibodies detected may be alloantibodies or autoantibodies to blood group antigens. The method described, therefore, provides improved methods of detecting and diagnosing diseases or a reaction to blood products involving the generation of alloantibodies or autoantibodies. Such diseases include haemolytic reactions to red cell transfusions (HTR), haemolytic disease of the newborn and autoimmune haemolytic anaemia.

Conveniently, the antibody is an alloantibody, allowing one to reliably and safely diagnose the risk of HTR or haemolytic disease of the newborn.

According to another aspect of the present invention there is provided a device for detecting the presence of an antibody in a sample to a blood group antigen. The device comprising a solid support (membrane, glass slide, microarray chip or bead(s) etc.) having an array of mimotopes of a blood group antigen immobilised thereon or having one mimotope on each solid support and having provided a plurality of solid supports.

An array may be constructed comprising only Rhesus, Kell, Duffy, Kidd, ABO, MNS, P, Lutheran, Lewis, Diego, Yt, Xg, Scianna, Dombrock, Colton, Landsteiner-Wiener, Chido-Rodgers, Hh, Kx, Gerbich, Cromer, Knops, Indian, Ok, Raph, JMH, I, Globoside or GIL antigen mimotopes. Alternatively, an array may be developed comprising any combination of blood group mimotopes, with mimotopes from multiple blood groups being arranged on the same diagnostic array. The composition and design of the array may depend on the intended purpose of the device and bespoke arrays may be developed as required. Arrays required for specific purposes can be constructed quickly, reliably and cost-effectively, without having to consider the availability of reagents.

With regard to the construction of arrays, different types and length of spacer for attaching peptides to a solid membrane support can be used, for example an amino-PEG spacer or a limited number of amino acid residues could be used to link the mimotope peptide sequence to the solid support.

Conveniently, the antibody is an alloantibody. The device can therefore be used for detecting alloantibodies in a sample of blood obtained from a patient suspected of generating alloantibodies to blood group antigens, without requiring the use of human blood group reagents. Removal of the need for blood group reagents will conveniently increase the availability of reagents for detection of antibodies in blood group samples.

Antigen-capture systems as described herein can also increase the sensitivity and accuracy of antibody detection, thus providing an improvement over existing methods for the detection of blood group.

By showing that phage-peptides, selected by biopanning, specifically bind the antibodies originally used to identify them, the experiments described herein confirm the potential value of the selected phage-peptides as diagnostic reagents.

The mimotopes identified in the present study, when coupled to a suitable solid phase, can be used as screening reagents to detect antibodies to blood group antigens.

In this respect, the mimotopes may be used to diagnose diseases associated with blood group antigens, such as diseases associated with the production of alloantibodies or autoantibodies.

Further potential uses of the mimotopes identified and described herein, together with uses of the arrays formed therewith, will be evident to a person skilled in the art.

The present invention will now be described by way of illustration only with reference to the accompanying drawings:

Figure 1 shows the results of phage ELISA, confirming binding of the anti-Fy^{b} antibody (LM447) to 14 of 20 Fy^{b} phage-peptides assayed.

Figure 2 shows the results of SPOTs hybridisation experiments. 20 Fyb peptide mimotopes were synthesised in duplicate. Binding experiments confirmed the ability of Fyb peptide mimotopes to recognise and bind the anti- Fy^{b} antibody, when synthesised on a membrane support.

Figure 3 shows the results of phage ELISA, confirming binding of the anti-Fy^{a} antibody (LM487) to 16 of 19 Fya phage-peptides assayed.

Figure 4 shows the results of a SPOTs hybridisation experiment. A series of 37 Fya peptide mimotopes were synthesised on a membrane and tested for their ability to bind the anti-Fy^{a} antibody (LM487).

Figure 5 shows the results of reciprocal hybridisation of Fya peptide mimotopes with the anti-Fy^{a} and anti-Fy^{b} monoclonal antibodies, LM447 and LM487. A series of Fy^{a} and peptide mimotopes were synthesised in duplicate and hybridisation of the membranes with the 2 monoclonals identified antibody-specific and cross-reactive peptide mimotopes.

Figure 6 shows the results of amino acid substitution experiments. A series of 16 synthetic peptides were synthesised in duplicate by SPOTs. These sequences were derived from the Fyb Consensus I sequence and generated by amino acid substitution. The synthetic peptide mimotopes were tested for their ability to bind the anti- Fy^{a} and anti-Fy^{b} monoclonal antibodies.

Figure 7 shows the results following hybridisation of a subset of the anti-RhD antibody-selected peptide mimotopes with the monoclonal antibody T10. 40 peptides (RhD41-RhD80) were synthesised and positive signals were observed in 11 spots (Spots 1-3, 8, 17, 27, 28, 31, 37, 39 & 40; see Table for peptide sequences).

Figure 8 shows the results of a SPOTs hybridisation experiment. A series of 28 RhD peptide mimotopes were synthesised by SPOTs and hybridisation of the membrane with the polyclonal anti-RhD antisera preparation identified antibody-reactive peptides.

Figure 9 shows the results of a SPOTs hybridisation experiment. A series of 36 RhE peptide mimotopes were synthesised by SPOTs. Hybridisation of the membrane with the monoclonal antibody E0002 identified antibody-specific peptide mimotopes.

Figure 10 shows the results of a SPOTs hybridisation experiment. A series of 16 Rhe peptide mimotopes were synthesised by SPOTs. Hybridisation of the membrane with the monoclonal antibody e0002 identified antibody-specific peptide mimotopes.

Figure 11 shows specific binding of anti-Fy^{b} monoclonal antibody (LM447) binding to peptide mimotope Fyb16 immobilised onto polystyrene LiquiChip Ni-NTA microsphere beads. The intensity of the signal equates to the quantity of monoclonal antibody bound to the immobilised peptide mimotope.

Figure 12 shows specific binding of anti-RhD monoclonal antibody (T27) binding to peptide mimotope RhD12 immobilised onto polystyrene LiquiChip Ni-NTA microsphere beads. The intensity of the signal equates to the quantity of monoclonal antibody bound to the immobilised peptide mimotope.

Figure 13 shows the specific binding of the anti-RhD monoclonal antibody (T27) to peptide mimotope RhD12 immobilised onto superparamagnetic polystyrene beads in a modified gel agglutination assay.Chamber 1: Beads + His-tagged peptide mimotope RhD12 only; Chamber 2: Beads + His-tagged RhD12 + anti-Fy^{b} monoclonal antibody (LM447); Chamber 3: Beads + His-tagged RhD12 + anti-RhD monoclonal antibody (T27).

Figure 14 shows the specific binding of the anti-Fy^{b} monoclonal antibody (LM447) to peptide mimotope Fyb16 immobilised onto superparamagnetic polystyrene beads in a modified gel agglutination assay. Chamber 1: Beads + His-tagged peptide mimotope Fyb16 only; Chamber 2: Beads + His-tagged Fyb16 + anti-Fy^{b} monoclonal antibody (LM447); Chamber 3: Beads + His-tagged Fyb16 + anti-RhD monoclonal antibody (T27) .

Examples

Methodology

Identification of mimotopes of blood group antigens commenced with biopanning of four phage-peptide libraries with a series of blood group antigen-specific monoclonal antibodies (3x anti-RhD, 3x RhE, 2x Rhe, 1x Fy^{a}, 1x Fy^{b}) and an RhD polyclonal preparation. In total, 490 phage were selected and initially characterised by DNA sequencing and ELISA. DNA sequence analysis identified 295 unique phage-peptide sequences, which were synthesised on membranes by SPOTS technology. The specificity of the peptide/antibody reaction was tested by ELISA and SPOTS assays and identified 84 peptides capable of detecting specific RhD, RhE, Rhe, Fy^{a} & Fy^{b} antibodies.

Biopanning

Three different phage libraries from New England Biolabs. (Ph.D. 7 and Ph.D. 12 displaying linear 7- and 12-mer peptides, respectively and Ph.D. C7C displaying 7-mer peptides constrained by a disulphide bridge at their termini) and a linear 15mer library (kindly provided by Prof. George P. Smith, University of Missouri) were used in biopanning experiments with 10 different monoclonal antibodies and a polyclonal preparation (Table 1).

Table 1: Antibodies Used in Biopanning.

| Specificity | Epitope | Class | ID | Source |
|---|---|---|---|---|
| Anti-RhD | epD3 [3.1] | IgGI/κ | T10 (LHM76/55) | Human |
| Anti-RhD | epD6/7[6.3] | IgGI/λ | T22 (LHM 169/80) | Human |
| Anti-RhD | epD1 [1.2] | IgGI/κ | T27 (LHM169/81) | Human |
| Anti-RhD | Polyclonal | - | Human Anti-D Ig | Human |
| Anti-RhE | Not known | IgG1 | E0001 (HIROI7) | Human |
| Anti-RhE | Not known | IgM | E0002 (NaTH110-1D6) | Human |
| Anti-RhE | Not known | IgM | E0003 (NaTH110-IH4) | Human |
| Anti-Rhe | Not known | IgM | e0002 (MS62) | Human |
| Anti-Rhe | Not known | IgM | e0003 (MS69) | Human |
| Anti-Fy^{a} | Not known | IgG1 | LM487 | Murine |
| Anti-Fy^{b} | Not known | IgG1 | LM447 | Murine |

All monoclonal antibodies listed were purified from culture supernatants. Polyclonal anti-RhD preparation was obtained from the Scottish National Blood Transfusion Service.

For each target antibody/library combination, a separate well of a 96-well Immulon 4 flat-bottomed ELISA plate (Thermo Labsystems) was coated with 5µg of antibody in 150µl ELISA coating buffer (15mM Na₂CO₃, 35mM NaHCO₃, pH9.6). Plates were sealed and incubated at 4°C overnight. Unbound antibody was discarded and 350µl of blocking buffer (0.1M NaHCO₃/3% skimmed milk powder) was added to each well. Plates were sealed and incubated for 1 hour at 4°C. The blocking buffer was removed and the wells were washed six times with 300µl TBS/0.1% Tween 20. Approximately 2x10¹¹ plaque forming units (pfu) in 100µl TBS/0.1% Tween 20 were added to each well and the plates were incubated at room temperature for 1 hour. Wells were washed ten times with 300µl TBS/0.1% Tween 20. Bound phage were eluted by incubation with 100µl of 0.2M glycine-HCl/BSA, pH2.2 (1mg/ml) for 10 minutes, then neutralised with 15µl of 1M Tris-HCL (pH9.1). An aliquot of the Round 1 eluate was removed and titred. The remainder of the eluted phage were amplified by infection of E. *coli* ER2538 cells for 4.5 hours at 37°C. The cultures were centrifuged for 10 minutes at 10,000 rpm at 4°C. The supernatants were recovered, 1/6 volume PEG/NaCl (20% polyethylene glycol 8000; 2.5M NaCl) added, then incubated overnight at 4°C. The PEG-precipitated phage were centrifuged at 10,000 rpm for 15 minutes at 4°C. The supernatant was removed and the phage pellet resuspended in 200µl TBS. Two further rounds of panning were undertaken and approximately 2 x 10¹¹ pfu of the Round 2 or Round 3 amplified eluates were used as input phage. Round 3 eluates were titrated but not amplified. Individual plaques were amplified and subjected to PCR amplification, prior to DNA sequence analysis. The specificity of peptide-antibody binding was subsequently determined using both phage ELISA and SPOTs techniques.

Phage Culture

Individual phage clones were randomly selected from the Round 3 titration plates and amplified in 1ml volumes of 1:100 dilution of an overnight culture of E. *coli* ER2538, grown in L-Broth. Cultures were incubated at 37°C for 4.5 hours, then centrifuged at 13000rpm for 1 minute. The phage supernatants were recovered for further analysis by PCR amplification, DNA sequencing and phage ELISA.

PCR Amplification

To determine the peptide sequences displayed in each of the selected phage clones, the DNA insert encoding the peptide sequence was obtained by PCR. PCR amplification was performed in a final volume of 25µl containing 1x buffer (Bioline), 400µM dNTPs, 1.5mM MgCl₂, 1 unit of Taq DNA polymerase (Bioline), 1µl of phage culture supernatant and 0.5µM each of the appropriate forward and reverse primers. The primers 12MER1For: 5-CGCAATTCCTTTAGTGGTAC-3 and 12MER2Rev: 5-CCCTCATAGTTAGCGTAACG-3 were used for amplification of the C7C, linear 7-mer and 12-mer phage/peptides. The primers Fuse5For1: 5'-ACCGATACAATTAAAGGCTC-3' and Fuse5Rev1: 5'-TGAATTTTCTGTATGAGG-3' were used for the amplification of the 15-mer phage/peptides. PCR was carried out in a thermal cycler (Techgene, model FTGene2D; Techne, Cambridge, UK) under the following conditions: denaturation at 95°C for 1 minute; annealing at 55°C for 1 minute; and extension at 72°C for 3 minutes. After 30 cycles, extension was continued at 72°C for an additional 9 minutes. PCR products were recovered using the QIAquick PCR Purification Kit (Qiagen) for sequence analysis.

Sequence Analysis

The nucleotide sequences of the phage peptides were obtained using the BigDye Terminator Cycle Sequencing Ready Reaction Kit and an ABI Prism 377 DNA Sequencer (Applied Biosystems). Primer 12MER2Rev was used to determine the random peptide sequences of the C7C, linear 7-mer and 12-mer PCR products. Primer Fuse5Rev1 was used for the 15-mer PCR products. Amino acid sequences were deduced from the nucleotide sequences using Lasergene software (DNASTAR Inc., Wisconsin, USA).

Phage ELISA

The ability of the recovered phage-peptides to react with the antibodies used in their selection was confirmed by capture ELISA. A 10µg/ml stock of appropriate monoclonal antibody was prepared in ELISA coating buffer and 150µl aliquots were added to wells of an Immulon 4 ELISA plate, and then incubated overnight at 4°C. Unbound antibody was discarded and the wells were blocked with 350µl of blocking buffer (0.1M NaHCO₃/3% skimmed milk powder) for 1 hour at 4°C. The wells were washed three times with 300µl TBS/0.5% Tween 20 and 100µl of selected phage culture supernatants were added to appropriate wells for 1 hour at room temperature. Unbound phage were removed and the wells washed six times with 300µl TBS/0.5% Tween 20. A 1:5000 dilution of horseradish peroxidase (HRP) conjugated anti-M13, secondary antibody was prepared in blocking buffer and 100µl aliquots added to each well. Following incubation for 1 hour at room temperature the wells were washed six times with 300µl TBS/0.5% Tween 20. To develop the reactions, a 200µl aliquot of o-phenylenediamine dihydrochloride (OPD) substrate (Sigma) was added to each well. The absorbance at 450nm was measured, following incubation at room temperature for 1 hour, by a microplate ELISA reader. An OD450 signal greater than 0.1 was considered positive.

SPOT Synthesis

The peptide mimotopes were synthesised by the SPOT method (Frank; 1992) using F-moc chemistry. The peptides were anchored to an amino-PEG derivatised cellulose membrane (Intavis AG) and synthesised as discrete spots. Synthesis was by stepwise elongation of the peptide chain from C-terminus to the N-terminus, by manual pipetting of the respective amino acids, according to the Sigma Genosys protocol. Incubation times were modified when using Intavis AG membranes. After addition of the final amino acid, the terminal residues and side chains of the peptides were deprotected and the membrane was washed with methanol; air dried and then stored at -20°C prior to use.

Screening of SPOT membranes

A membrane was placed in a 50ml Falcon tube and blocked overnight at 4°C with gentle agitation in TBS/0.25% Tween 20/5% skimmed milk powder. A 2µg/ml dilution of the primary (1°) antibody (monoclonal or polyclonal) was prepared in TBS/0.25% Tween20/5% skimmed milk powder before incubation with the membrane for 1 hour at room temperature on a spiral rotor. Following 4 washes, each for 5 minutes with 20ml of TBS/0.25% Tween20, the membrane was incubated for 1 hour at room temperature with a 1/5000 dilution of the secondary (2°) antibody, prepared in TBS/0.25% Tween20/5% skimmed milk powder. Peroxidase labelled rabbit anti-mouse IgG (Sigma) was used for the detection of the murine anti-Fy^{a} (LM487) and anti-Fy^{b} (LM447) 1° antibodies and peroxidase labelled goat anti-human IgG (Pharmacia) for the detection of the human 1° antibodies. Following 4 washes, each for 5 minutes with 20ml of TBS/0.25% Tween20, the membrane was blotted to remove excess fluid. Bound antibody was detected by chemiluminescence. ECL detection reagents (Amersham Biosciences) were mixed and overlaid on the membrane for 2 minutes. The chemiluminescent substrate was removed and the membrane blotted to remove excess fluid. Positive spots were visualised following exposure to Hyperfilm ECL (Amersham Biosciences) for a series of short incubation times (5 seconds to 10 minutes).

Example 1

Identification of phage-peptides which mimic the Duffy antigenic epitopes

The anti-Fy^{a} (LM487) and anti-Fy^{b} (LM447) -specific monoclonal antibodies were developed using immunogenic peptides derived from epitopes on the Duffy glycoprotein (Colligan et al, 1998). These antibodies were used to pan phage libraries for peptides that mimicked the native Duffy antigens. Clones from the final third round of each panning experiment were chosen at random for amplification and DNA sequencing. The amino acid sequences encoded by the phage-peptides were deduced from the DNA sequence data.

Biopanning of each of the 4 phage-peptide libraries was undertaken with the anti-Fy^{b} monoclonal antibody (LM447) and resulted in the identification of multiple copies of particular sequences from each of the libraries. In total, 100 unique Fyb peptide mimotopes were identified (see Table 2/ peptides Fyb1-71 & Fyb88-116). None of the sequences identified resembled the 14-mer immunisation peptide (PDGDYDANLEAAAP; CPep75) used in the development of the LM447 antibody. However, analysis of the amino acid sequences of these "Fyb" peptide mimotopes identified 2 consensus sequences:

Consensus 1: **M**(F/Y)**QPD**(N/P) (P/L) (T/P)**T**(K/L) (N/Q) (P/V/A/S)

Consensus 2: **D** (H/M/V) **HYT** (S/N) **NTDPL** (H/N/R) (A/P/V) **P**

Certain positions within each consensus sequence had a limited number of amino acids (shown in brackets). These occurred with equal frequency amongst the total population of Fyb peptide mimotopes selected.

Table 2: Fyb peptides

| SEQ ID NO | Pep ID | Panning Antibody | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|---|---|
| 1 | Fy b1 | Fy^{b} | M | Y | Q | D | T | P | P | P | R | K | N | V | | | |
| 2 | Fy b2 | Fy^{b} | M | Y | Q | P | G | P | Q | G | L | P | Q | S | | | |
| 3 | Fy b3 | Fy^{b} | M | Y | Q | P | P | S | T | T | R | M | Y | P | | | |
| 4 | Fy b4 | Fy^{b} | M | Y | Q | D | A | G | P | T | E | W | S | Y | | | |
| 5 | Fy b5 | Fy^{b} | M | W | Q | P | A | P | P | S | V | R | G | A | | | |
| 6 | Fy b6 | Fy^{b} | M | F | Q | S | A | G | Y | P | S | S | F | R | | | |
| 7 | Fy b7 | Fy^{b} | M | F | Q | E | H | T | P | A | F | G | G | L | | | |
| 8 | Fy b8 | Fy^{b} | M | F | Q | E | P | L | P | P | F | K | A | S | | | |
| 9 | Fy b9 | Fy^{b} | M | F | Q | P | T | H | W | G | T | R | L | S | | | |
| 10 | Fy b10 | Fy^{b} | M | Q | Q | P | A | G | W | K | Y | L | G | Q | | | |
| 11 | Fy b11 | Fy^{b} | M | V | Q | E | L | P | F | V | L | R | S | K | | | |
| 12 | Fy b12 | Fy^{b} | T | F | Q | P | R | S | W | P | N | I | D | L | | | |
| 13 | Fy b13 | Fy^{b} | T | F | Q | Y | L | D | P | P | P | F | T | P | | | |
| 14 | Fy b14 | Fy^{b} | T | Y | Q | H | N | N | P | D | A | F | R | L | | | |
| 15 | Fy b15 | Fy^{b} | M | F | Q | S | N | T | Y | P | I | W | N | V | | | |
| 16 | Fy b16 | Fy^{b} | F | Q | P | N | P | P | P | S | F | A | A | P | | | |
| 17 | Fy b17 | Fy^{b} | M | Y | Q | G | R | N | D | P | H | L | L | P | | | |
| 18 | Fy b18 | Fy^{b} | M | Y | Q | P | R | D | W | R | S | A | P | P | | | |
| 19 | Fy b19 | Fy^{b} | M | F | N | A | D | P | T | I | P | A | Q | P | | | |
| 20 | Fy b20 | Fy^{b} | H | G | T | N | N | T | D | | | | | | | | |
| 21 | Fy b21 | Fy^{b} | Q | L | D | N | L | T | T | | | | | | | | |
| 22 | Fy b22 | Fy^{b} | H | Y | Y | H | N | T | D | | | | | | | | |
| 23 | Fy b23 | Fy^{b} | H | A | L | P | L | H | A | | | | | | | | |
| 24 | Fy b24 | Fy^{b} | M | F | Q | E | H | P | I | | | | | | | | |
| 25 | Fy b25 | Fy^{b} | M | W | Q | P | S | T | F | | | | | | | | |
| 26 | Fy b26 | Fy^{b} | M | F | Q | P | A | V | Q | | | | | | | | |
| 27 | Fy b27 | Fy^{b} | M | Y | Q | P | L | A | L | | | | | | | | |
| 28 | Fy b28 | Fy^{b} | M | F | Q | P | D | F | L | | | | | | | | |
| 29 | Fy b29 | Fy^{b} | M | F | Q | E | L | V | I | | | | | | | | |
| 30 | Fy b30 | Fy^{b} | M | F | Q | A | N | T | V | | | | | | | | |
| 31 | Fy b31 | Fy^{b} | M | F | Q | P | L | H | R | | | | | | | | |
| 32 | Fy b32 | Fy^{b} | M | F | Q | P | T | D | H | | | | | | | | |
| 33 | Fy b33 | Fy^{b} | M | F | Q | P | L | P | S | | | | | | | | |
| 34 | Fy b34 | Fy^{b} | M | F | Q | P | Q | Y | H | | | | | | | | |
| 35 | Fy b35 | Fy^{b} | M | F | Q | P | P | L | K | | | | | | | | |
| 36 | Fy b36 | Fy^{b} | M | Y | Q | P | P | G | S | | | | | | | | |
| 37 | Fy b37 | Fy^{b} | M | Y | Q | P | R | T | L | | | | | | | | |
| 38 | Fy b38 | Fy^{b} | M | W | Q | P | D | A | R | | | | | | | | |
| 39 | Fy b39 | Fy^{b} | M | Y | Q | D | L | P | Y | | | | | | | | |
| 40 | Fy b40 | Fy^{b} | M | W | Q | P | Q | P | M | P | G | M | V | G | | | |
| 41 | Fy b41 | Fy^{b} | M | Y | Q | P | R | N | W | S | D | A | S | G | | | |
| 42 | Fy b42 | Fy^{b} | T | Y | Q | P | K | S | P | S | N | F | A | N | | | |
| 43 | Fy b43 | Fy^{b} | M | F | Q | P | N | N | P | P | G | I | H | A | | | |
| 44 | Fy b44 | Fy^{b} | M | F | Q | P | Y | H | P | | | | | | | | |
| 45 | Fy b45 | Fy^{b} | M | Y | N | P | P | W | G | | | | | | | | |
| 46 | Fy b46 | Fy^{b} | T | Y | Q | P | L | T | W | | | | | | | | |
| 47 | Fy b47 | Fy^{b} | V | S | H | T | N | N | T | D | L | R | P | P | | | |
| 48 | Fy b48 | Fy^{b} | D | H | P | P | T | L | N | R | P | P | N | V | | | |
| 49 | Fy b49 | Fy^{b} | H | G | V | S | N | T | D | | | | | | | | |
| 50 | Fy b50 | Fy^{b} | M | W | Q | P | R | T | M | | | | | | | | |
| 51 | Fy b51 | Fy^{b} | H | Y | H | S | N | T | D | | | | | | | | |
| 52 | Fy b52 | Fy^{b} | H | Y | A | S | N | T | D | | | | | | | | |
| 53 | Fy b53 | Fy^{b} | M | Y | Q | P | M | T | A | | | | | | | | |
| 54 | Fy b54 | Fy^{b} | M | Y | Q | P | N | S | T | T | Q | L | Y | N | | | |
| 55 | Fy b55 | Fy^{b} | N | F | Q | P | N | D | P | P | A | R | P | S | | | |
| 56 | Fy b56 | Fy^{b} | T | Y | Q | H | M | P | P | P | H | S | P | F | | | |
| 57 | Fy b57 | Fy^{b} | M | Y | Q | E | S | A | P | P | R | Q | V | F | | | |
| 58 | Fy b58 | Fy^{b} | M | W | Q | P | N | S | P | S | V | P | P | P | | | |
| 59 | Fy b59 | Fy^{b} | I | F | N | D | P | W | M | | | | | | | | |
| 60 | Fy b60 | Fy^{b} | M | F | Q | P | L | P | A | | | | | | | | |
| 61 | Fy b61 | Fy^{b} | M | Y | Q | P | T | H | W | | | | | | | | |
| 62 | Fy b62 | Fy^{b} | M | F | Q | P | T | L | V | A | P | L | Q | A | | | |
| 63 | Fy b63 | Fy^{b} | M | F | Q | S | S | P | P | P | L | G | H | A | | | |
| 64 | Fy b64 | Fy^{b} | T | F | Q | P | R | L | F | S | S | W | V | H | | | |
| 65 | Fy b65 | Fy^{b} | M | F | Q | S | R | G | P | N | Q | L | F | P | | | |
| 66 | Fy b66 | Fy^{b} | M | F | Q | P | A | N | P | S | Q | S | T | A | | | |
| 67 | Fy b67 | Fy^{b} | M | W | Q | P | Q | W | T | | | | | | | | |
| 68 | Fy b68 | Fy^{b} | M | F | Q | P | P | R | N | | | | | | | | |
| 69 | Fy b69 | Fy^{b} | M | F | Q | P | R | F | P | | | | | | | | |
| 70 | Fy b70 | Fy^{b} | M | F | Q | P | V | E | L | | | | | | | | |
| 71 | Fy b71 | Fy^{b} | M | Y | Q | P | N | L | M | | | | | | | | |
| 72 | Fy b72 | Fy^{b} | M | Y | Q | P | D | P | P | P | T | K | N | P | | | |
| 73 | Fy b73 | Fy^{b} | M | Y | Q | P | D | P | P | P | T | K | N | V | | | |
| 74 | Fy b74 | Fy^{b} | M | Y | Q | P | D | P | P | T | T | K | N | P | | | |
| 75 | Fy b75 | Fy^{b} | M | Y | Q | P | D | P | P | T | T | K | N | V | | | |
| 76 | Fy b76 | Fy^{b} | M | Y | Q | P | D | N | P | P | T | K | N | P | | | |
| 77 | Fy b77 | Fy^{b} | M | Y | Q | P | D | N | P | P | T | K | N | V | | | |
| 78 | Fy b78 | Fy^{b} | M | Y | Q | P | D | N | P | T | T | K | N | P | | | |
| 79 | Fy b79 | Fy^{b} | M | Y | Q | P | D | N | P | T | T | K | N | V | | | |
| 80 | Fy b80 | Fy^{b} | M | F | Q | P | D | P | P | P | T | K | N | P | | | |
| 81 | Fy b81 | Fy^{b} | M | F | Q | P | D | P | P | P | T | K | N | V | | | |
| 82 | Fy b82 | Fy^{b} | M | F | Q | P | D | P | P | T | T | K | N | P | | | |
| 83 | Fy b83 | Fy^{b} | M | F | Q | P | D | P | P | T | T | K | N | V | | | |
| 84 | Fy b84 | Fy^{b} | M | F | Q | P | D | N | P | P | T | K | N | P | | | |
| 85 | Fy b85 | Fy^{b} | M | F | Q | P | D | N | P | P | T | K | N | V | | | |
| 86 | Fy b86 | Fy^{b} | M | F | Q | P | D | N | P | T | T | K | N | P | | | |
| 87 | Fy b87 | Fy^{b} | M | F | Q | P | D | N | P | T | T | K | N | V | | | |
| 88 | Fy b88 | Fy^{b} | S | P | P | T | P | P | R | F | S | D | D | W | | | |
| 89 | Fy b89 | Fy^{b} | L | P | T | L | H | T | A | H | P | P | V | Q | | | |
| 90 | Fy b90 | Fy^{b} | G | T | Q | T | R | S | S | Y | V | L | T | S | | | |
| 91 | Fy b91 | Fy^{b} | N | P | H | P | M | W | N | P | T | S | Y | L | | | |
| 92 | Fy b92 | Fy^{b} | A | Y | R | A | V | T | L | N | Q | P | L | T | | | |
| 93 | Fy b93 | Fy^{b} | Q | Y | L | P | D | H | R | S | S | Q | P | N | | | |
| 94 | Fy b94 | Fy^{b} | L | P | H | R | T | D | Y | L | Y | T | P | E | | | |
| 95 | Fy b95 | Fy^{b} | H | P | A | V | Q | P | P | L | I | Y | M | F | | | |
| 96 | Fy b96 | Fy^{b} | V | P | T | Y | E | I | I | A | R | G | I | R | | | |
| 97 | Fy b97 | Fy^{b} | S | P | S | D | M | F | I | S | T | Q | R | L | | | |
| 98 | Fy b98 | Fy^{b} | L | L | T | Q | T | T | A | Y | A | P | M | S | | | |
| 99 | Fy b99 | Fy^{b} | M | M | N | S | D | P | K | S | F | L | S | L | | | |
| 100 | Fy b100 | Fy^{b} | M | R | N | D | V | P | W | I | P | W | P | V | | | |
| 101 | Fy b101 | Fy^{b} | T | L | P | Y | L | K | M | H | R | N | L | L | | | |
| 102 | Fy b102 | Fy^{b} | Q | S | Y | S | P | R | F | | | | | | | | |
| 103 | Fy b103 | Fy^{b} | L | K | Q | R | T | L | M | | | | | | | | |
| 104 | Fy b104 | Fy^{b} | Q | S | Y | P | Q | A | L | | | | | | | | |
| 105 | Fy b105 | Fy^{b} | Q | S | Y | S | P | K | Y | | | | | | | | |
| 106 | Fy b106 | Fy^{b} | A | M | Y | Q | P | T | H | | | | | | | | |
| 107 | Fy b107 | Fy^{b} | H | W | Y | H | N | T | D | | | | | | | | |
| 108 | Fy b108 | Fy^{b} | L | S | Y | S | P | R | Y | | | | | | | | |
| 109 | Fy b109 | Fy^{b} | E | S | Y | S | P | R | H | | | | | | | | |
| 110 | Fy b110 | Fy^{b} | Q | S | Y | P | S | V | Y | | | | | | | | |
| 111 | Fy b111 | Fy^{b} | D | S | Y | S | P | K | F | | | | | | | | |
| 112 | Fy b112 | Fy^{b} | Q | S | Y | P | A | K | F | | | | | | | | |
| 113 | Fy b113 | Fy^{b} | R | S | A | S | A | L | W | | | | | | | | |
| 114 | Fy b114 | Fy^{b} | E | S | Y | S | P | R | L | | | | | | | | |
| 115 | Fy b115 | Fy^{b} | P | I | F | T | K | L | H | | | | | | | | |
| 116 | Fy b116 | Fy^{b} | Q | S | Y | P | Y | R | G | | | | | | | | |

Biopanning with the anti-Fy^{a} monoclonal, LM487, identified a total of 37 unique peptide sequences (see Table 3/Fy^{a} peptides Fya1-37). A small number of the "Fya" phage-) peptides, contained sequence identical to the Fy^{a} polymorphism, present within the original 14-mer immunisation peptide (PDGDYGANLEAAAP; CPep118), thus validating the use of phage display as an approach to identifying blood group antigen mimics. Sequence analysis of the amino acid sequences of these "Fya" peptide mimotopes also identified 2 putative consensus sequences:

Consensus I: **YNYQSYPNPFPV**

Consensus II: **GI** (A/S) (E/D) (D/G)**DYGALSW**

However, in contrast to the "Fyb" results, many of these "Fya" peptide mimotope sequences did not conform to either consensus sequence identified above.

Table 3: Fya peptides

| SEQ ID NO | Pep ID | Panning Antibody | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|---|---|
| 117 | Fy a1 | Fy^{a} | W | Q | S | Y | P | N | P | | | | | | | | |
| 118 | Fy a2 | Fy^{a} | T | N | L | R | L | P | A | | | | | | | | |
| 119 | Fy a3 | Fy^{a} | Y | Q | S | Y | P | P | P | | | | | | | | |
| 120 | Fy a4 | Fy^{a} | Y | Q | S | Y | P | M | R | | | | | | | | |
| 121 | Fy a5 | Fy^{a} | M | Y | P | D | W | Q | Q | | | | | | | | |
| 122 | Fy a6 | Fy^{a} | L | E | R | D | P | P | V | | | | | | | | |
| 123 | Fy a7 | Fy^{a} | Y | Q | S | Y | P | N | W | | | | | | | | |
| 124 | Fy a8 | Fy^{a} | L | V | P | P | D | G | Y | | | | | | | | |
| 125 | Fy a9 | Fy^{a} | A | P | F | W | Q | W | M | | | | | | | | |
| 126 | Fy a10 | Fy^{a} | F | S | P | P | N | W | Y | | | | | | | | |
| 127 | Fy a11 | Fy^{a} | F | A | P | W | Y | Q | Q | R | | | | | | | |
| 128 | Fy a12 | Fy^{a} | Q | S | Y | P | N | T | F | | | | | | | | |
| 129 | Fy a13 | Fy^{a} | D | W | L | F | K | M | S | | | | | | | | |
| 130 | Fy a14 | Fy^{a} | G | D | W | L | H | W | | | | | | | | | |
| 131 | Fy a15 | Fy^{a} | S | D | G | D | Y | G | A | | | | | | | | |
| 132 | Fy a16 | Fy^{a} | G | P | S | W | Q | S | T | | | | | | | | |
| 133 | Fy a17 | Fy^{a} | V | P | Y | P | N | Q | M | C | | | | | | | |
| 134 | Fy a18 | Fy^{a} | P | S | P | G | Q | I | | | | | | | | | |
| 135 | Fy a19 | Fy^{a} | F | E | P | P | N | W | D | S | G | P | R | P | | | |
| 136 | Fy a20 | Fy^{a} | G | I | A | E | D | D | Y | G | A | L | S | W | | | |
| 137 | Fy a21 | Fy^{a} | Y | N | Q | Y | Q | S | Y | P | P | S | L | Q | | | |
| 138 | Fy a22 | Fy^{a} | Y | Q | S | Y | P | S | R | P | P | V | R | L | | | |
| 139 | Fy a23 | Fy^{a} | A | L | A | P | E | E | D | E | V | Y | Y | V | | | |
| 140 | Fy a24 | Fy^{a} | V | L | L | P | E | S | D | E | S | H | R | A | | | |
| 141 | Fy a25 | Fy^{a} | I | P | N | W | Q | S | C | | | | | | | | |
| 142 | Fy a26 | Fy^{a} | S | G | V | A | F | C | P | P | W | W | C | D | G | P | L |
| 143 | Fy a27 | Fy^{a} | W | P | R | H | V | P | L | F | G | L | D | G | Y | V | T |
| 144 | Fy a28 | Fy^{a} | A | R | E | Y | G | T | R | F | S | L | I | G | G | Y | R |
| 145 | Fy a29 | Fy^{a} | A | S | R | R | L | L | P | S | D | V | R | L | P | S | S |
| 146 | Fy a30 | Fy^{a} | L | W | D | P | P | P | F | G | L | S | R | I | F | F | G |
| 147 | Fy a31 | Fy^{a} | D | A | R | P | L | A | W | Y | E | E | P | S | F | W | M |
| 148 | Fy a32 | Fy^{a} | S | G | Y | A | R | P | F | Y | Q | S | Y | P | A | A | S |
| 149 | Fy a33 | Fy^{a} | V | N | S | T | K | W | P | G | M | P | S | F | | | |
| 150 | Fy a34 | Fy^{a} | V | S | P | P | E | W | Y | P | L | A | A | D | | | |
| 151 | Fy a35 | Fy^{a} | T | G | P | K | L | H | C | P | P | A | V | C | | | |
| 152 | Fy a36 | Fy^{a} | F | K | N | P | S | Q | S | Y | P | P | E | P | | | |
| 153 | Fy a37 | Fy^{a} | F | T | M | E | R | D | P | P | I | A | R | V | | | |

The data shows that phage-peptides were identified that mimicked the Duffy antigenic epitopes. The anti-Fy^{a} antibody bound phage-peptides (Peptides Fya15 & Fya20) that contained sequences identical to the Fy^{a} polymorphism and the anti-Fy^{b} antibody selected peptide mimotopes, confirming the use of phage display as a valid means of identifying blood group antigen mimics.

Example 2

Identification of phage-peptides which mimic the complex RhD antigenic epitopes

Phage-peptides that mimic RhD antigenic epitopes were identified with 3 monoclonal anti-Ds (T10, T22 & T27). Biopanning of the 4 phage-peptide libraries with this limited number of monoclonal antibodies selected 80 RhD peptide mimotopes (see Table 4/ Peptides RhD 1-80). Additionally, biopanning with a polyclonal anti-RhD preparation identified a further 25 RhD peptide mimotopes (see Table 4/ peptides RhD81-105). Peptide RhD52 was selected with both T10 and the polyclonal anti-RhD preparation.

Table 4: RhD peptides

| SEQ ID NO | Pep ID | Panning Antibody | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|---|---|
| 154 | RhD 1 | RhD T27 | G | L | F | K | I | Q | P | | | | | | | | |
| 155 | RhD 2 | RhD T27 | L | G | H | H | N | P | H | | | | | | | | |
| 156 | RhD 3 | RhD T27 | N | L | L | P | A | K | L | | | | | | | | |
| 157 | RhD 4 | RhD T27 | H | T | A | L | S | T | S | | | | | | | | |
| 158 | RhD 5 | RhD T27 | T | P | G | S | G | Q | F | | | | | | | | |
| 159 | RhD 6 | RhD T27 | F | S | P | N | N | S | E | T | T | N | Y | T | | | |
| 160 | RhD 7 | RhD T27 | T | T | V | P | D | T | W | V | L | F | P | R | | | |
| 161 | RhD 8 | RhD T27 | S | N | V | P | L | H | W | I | L | S | P | R | | | |
| 162 | RhD 9 | RhD T27 | W | H | F | E | W | W | R | A | T | P | S | G | | | |
| 163 | RhD 10 | RhD T27 | N | Y | P | A | V | V | S | N | S | L | L | A | | | |
| 164 | RhD 11 | RhD T27 | D | V | Y | D | T | R | P | Y | H | G | F | M | | | |
| 165 | RhD 12 | RhD T27 | H | Q | N | P | W | L | L | W | P | R | G | F | | | |
| 166 | RhD 13 | RhD T27 | V | I | D | W | L | L | C | P | R | G | F | S | A | C | M |
| 167 | RhD 14 | RhD T27 | D | R | R | I | F | W | W | S | L | R | S | A | P | G | A |
| 168 | RhD 15 | RhD T27 | L | H | A | W | H | R | N | T | I | G | W | W | I | G | F |
| 169 | RhD 16 | RhD T22 | W | G | L | W | F | S | N | | | | | | | | |
| 170 | RhD 17 | RhD T22 | W | G | I | W | W | N | N | | | | | | | | |
| 171 | RhD 18 | RhD T22 | W | G | L | W | S | T | P | | | | | | | | |
| 172 | RhD 19 | RhD T22 | W | V | H | L | Q | S | P | | | | | | | | |
| 173 | RhD 20 | RhD T22 | W | G | L | W | W | H | T | | | | | | | | |
| 174 | RhD 21 | RhD T22 | W | G | L | W | W | T | G | | | | | | | | |
| 175 | RhD 22 | RhD T22 | L | L | G | L | T | E | T | | | | | | | | |
| 176 | RhD 23 | RhD T22 | D | S | R | W | L | L | P | | | | | | | | |
| 177 | RhD 24 | RhD T22 | F | H | W | W | P | R | T | Q | D | P | H | R | | | |
| 178 | RhD 25 | RhD T22 | F | H | W | S | W | Y | T | P | S | R | P | S | | | |
| 179 | RhD 26 | RhD T22 | H | S | S | W | W | L | A | L | A | K | P | T | | | |
| 180 | RhD 27 | RhD T22 | W | H | W | Y | P | R | F | S | P | P | S | H | | | |
| 181 | RhD 28 | RhD T22 | N | D | Y | S | D | S | N | Q | V | P | A | S | | | |
| 182 | RhD 29 | RhD T22 | F | H | W | P | R | S | W | V | T | W | Q | s | | | |
| 183 | RhD 30 | RhD T22 | H | L | S | S | R | H | L | F | V | P | L | S | | | |
| 184 | RhD 31 | RhD T22 | T | I | T | D | C | Y | P | I | C | V | S | P | | | |
| 185 | RhD 32 | RhD T22 | W | P | C | H | P | I | C | L | S | P | R | G | | | |
| 186 | RhD 33 | RhD T22 | A | M | D | C | F | P | I | C | L | W | N | P | | | |
| 187 | RhD 34 | RhD T22 | W | N | C | F | P | I | C | H | A | S | G | L | | | |
| 188 | RhD 35 | RhD T22 | V | Y | A | L | G | C | W | P | I | C | H | K | | | |
| 189 | RhD 36 | RhD T22 | H | H | V | F | T | P | N | C | Y | P | I | C | | | |
| 190 | RhD 37 | RhD T22 | W | H | W | S | S | L | S | W | P | A | L | P | | | |
| 191 | RhD 38 | RhD T22 | G | N | W | L | F | N | S | C | Y | P | L | C | F | P | L |
| 192 | RhD 39 | RhD T22 | A | F | L | G | H | S | H | W | F | P | S | V | A | S | R |
| 193 | RhD 40 | RhD T22 | Q | L | L | C | F | P | I | C | R | P | E | P | P | V | S |
| 194 | RhD 41 | RhD T10 | W | Y | T | K | A | P | Y | | | | | | | | |
| 195 | RhD 42 | RhD T10 | W | Y | H | K | A | P | Y | | | | | | | | |
| 196 | RhD 43 | RhD T10 | Y | Y | Q | R | A | P | Y | | | | | | | | |
| 197 | RhD 44 | RhD T10 | H | W | K | H | P | W | G | A | W | D | T | L | | | |
| 198 | RhD 45 | RhD T10 | W | H | W | Q | W | T | P | W | S | I | Q | P | | | |
| 199 | RhD 4 6 | RhD T10 | W | H | K | N | W | W | P | P | S | T | P | N | | | |
| 200 | RhD 47 | RhD T10 | S | M | S | S | M | L | L | A | A | Q | T | V | | | |
| 201 | RhD 48 | RhD T10 | Q | S | H | Y | R | H | I | S | P | A | Q | V | | | |
| 202 | RhD 49 | RhD T10 | S | M | S | Q | P | K | S | Q | V | N | A | H | | | |
| 203 | RhD 50 | RhD T10 | W | H | W | T | F | Y | T | P | L | E | S | T | | | |
| 204 | RhD 51 | RhD T10 | E | S | L | S | T | D | T | Y | A | I | L | L | | | |
| 205 | RhD 52 | RhD T10 | H | S | S | W | Y | I | Q | H | F | P | P | L | | | |
| 206 | RhD 53 | RhD T10 | S | T | Y | L | N | G | P | T | G | V | D | L | | | |
| 207 | RhD 54 | RhD T10 | Q | H | K | T | S | I | T | G | H | L | E | P | | | |
| 208 | RhD 55 | RhD T10 | F | H | R | W | P | T | W | P | L | P | S | P | | | |
| 209 | RhD 56 | RhD T10 | V | P | P | W | V | S | V | R | T | G | P | G | | | |
| 210 | RhD 57 | RhD T10 | T | L | V | Y | Q | P | P | W | Y | R | I | A | | | |
| 211 | RhD 58 | RhD T10 | F | H | Q | R | L | W | W | P | T | H | T | P | | | |
| 212 | RhD 59 | RhD T10 | W | H | W | R | L | Y | S | A | N | T | P | | | | |
| 213 | RhD 60 | RhD T10 | H | A | A | F | E | P | R | G | D | V | R | H | T | L | L |
| 214 | RhD 61 | RhD T10 | S | I | W | D | L | P | L | Q | Y | R | G | F | G | T | S |
| 215 | RhD 62 | RhD T10 | L | W | R | L | R | G | G | S | F | P | V | I | S | H | G |
| 216 | RhD 63 | RhD T10 | R | N | A | L | H | S | L | R | T | L | S | S | S | W | V |
| 217 | RhD 64 | RhD T10 | F | H | R | H | W | W | P | P | T | L | S | T | | | |
| 218 | RhD 65 | RhD T10 | R | P | H | L | L | D | W | E | L | N | P | V | | | |
| 219 | RhD 66 | RhD T10 | F | H | W | R | W | S | T | F | P | E | Y | P | | | |
| 220 | RhD 67 | RhD T10 | V | W | A | V | S | L | P | W | Y | R | Y | P | | | |
| 221 | RhD 68 | RhD T10 | L | D | T | Y | W | Y | R | E | H | F | R | R | | | |
| 222 | RhD 69 | RhD T10 | V | H | W | R | W | W | D | Q | R | V | P | M | | | |
| 223 | RhD 70 | RhD T10 | L | P | W | Y | Q | L | T | | | | | | | | |
| 224 | RhD 71 | RhD T10 | V | P | W | F | R | A | P | | | | | | | | |
| 225 | RhD 72 | RhD T10 | W | H | P | P | Q | P | S | | | | | | | | |
| 226 | RhD 73 | RhD T10 | F | H | E | N | W | P | S | | | | | | | | |
| 227 | RhD 74 | RhD T10 | F | W | W | Q | V | P | A | | | | | | | | |
| 228 | RhD 75 | RhD T10 | T | Q | W | Y | Q | I | A | | | | | | | | |
| 229 | RhD 7 6 | RhD T10 | L | P | W | F | Q | L | P | | | | | | | | |
| 230 | RhD 77 | RhD T10 | T | P | L | S | K | S | T | | | | | | | | |
| 231 | RhD 78 | RhD T10 | L | P | W | Y | A | T | P | | | | | | | | |
| 232 | RhD 79 | RhD T10 | L | P | W | Y | R | H | | | | | | | | | |
| 233 | RhD 80 | RhD T10 | I | P | W | Y | K | I | T | | | | | | | | |
| 234 | RhD 81 | RhD polyclonal | F | H | S | T | W | P | W | R | E | A | E | G | | | |
| 235 | RhD 82 | RhD polyclonal | F | H | A | N | W | P | Q | S | A | R | D | V | | | |
| 236 | RhD 83 | RhD polyclonal | F | H | S | D | W | P | G | Q | T | F | T | W | | | |
| 237 | RhD 84 | RhD polyclonal | F | H | E | N | W | S | T | R | P | T | T | R | | | |
| 238 | RhD 85 | RhD polyclonal | F | H | S | V | Y | P | W | R | E | A | E | G | | | |
| 239 | RhD 8 6 | RhD polyclonal | F | H | S | N | W | P | S | A | Y | T | A | R | | | |
| 240 | RhD 87 | RhD polyclonal | F | H | S | N | W | P | S | L | I | R | A | R | | | |
| 241 | RhD 88 | RhD polyclonal | A | G | Y | Q | I | G | M | P | N | P | L | L | | | |
| 242 | RhD 89 | RhD polyclonal | F | H | W | R | Y | P | L | P | L | P | G | Q | | | |
| 243 | RhD 90 | RhD polyclonal | S | P | T | S | F | R | Q | V | F | G | F | Y | | | |
| 244 | RhD 91 | RhD polyclonal | A | L | P | E | L | S | S | L | P | E | S | A | R | | |
| 245 | RhD 92 | RhD polyclonal | L | H | W | W | P | T | Y | G | N | N | G | M | | | |
| 246 | RhD 93 | RhD polyclonal | F | H | R | P | Y | Y | W | P | P | T | P | L | | | |
| 247 | RhD 94 | RhD polyclonal | F | H | W | R | L | P | Y | P | L | P | S | S | | | |
| 248 | RhD 95 | RhD polyclonal | I | H | W | W | V | K | S | P | P | P | G | S | | | |
| 249 | RhD 96 | RhD polyclonal | S | H | W | W | T | S | I | L | A | T | P | S | | | |
| 250 | RhD 97 | RhD polyclonal | F | H | W | H | L | Q | P | Q | L | W | S | Y | | | |
| 251 | RhD 98 | RhD polyclonal | L | H | R | E | W | I | Y | P | Y | L | I | S | | | |
| 252 | RhD 99 | RhD polyclonal | G | Q | K | T | H | N | P | F | H | L | H | P | | | |
| 253 | RhD 100 | RhD polyclonal | A | T | W | S | H | H | L | S | S | A | G | L | | | |
| 254 | RhD 101 | RhD polyclonal | F | H | R | H | Y | Y | P | W | A | L | I | Q | | | |
| 255 | RhD 102 | RhD polyclonal | H | S | L | K | H | T | Q | M | S | Y | S | S | | | |
| 256 | RhD 103 | RhD polyclonal | S | V | S | V | G | M | K | P | S | P | R | P | | | |
| 257 | RhD 104 | RhD polyclonal | W | P | H | Q | V | H | K | H | I | Y | R | Q | | | |
| 258 | RhD 105 | RhD polyclonal | A | P | P | Y | P | G | P | L | P | L | S | L | | | |

Example 3

Identification of phage-peptides which mimic RhEe antigenic epitopes

Biopanning was undertaken with 3 anti-RhE monoclonal antibodies (1x IgG, 2x IgM; see Table 1) and resulted in the identification of multiple copies of many sequences from each of the 4 phage-peptide libraries. Sequence similarities were also evident between the selected phage-peptides from the different libraries. Peptides RhE18 and RhE24 were selected with antibodies E0002 and E0003 and Peptide RhE19 was selected with antibodies E0001 and E0002 These results indicate that, regardless of class, antibodies raised against the same epitope will identify some phage with the identical peptide sequence. In total, 36 unique RhE peptide mimotopes were identified (see Table 5; Peptides RhE1-36). Biopanning with 2 anti-Rhe monoclonal antibodies (2x IgM; see Table 1) identified 17 unique Rhe peptide mimotopes (see Table 5; Peptides Rhe1-17).

Table 5: RhE/e

| | | | | | | | | | | | | | | | | | |
|---|---|---|---|---|---|---|---|---|---|---|---|---|---|---|---|---|---|
| SEQ ID NO | Pep ID | Panning Antibody | 1 | 2 | 3 | 4 | 5 | 6 | 7 | 8 | 9 | 10 | 11 | 12 | 13 | 14 | 15 |
| 259 | RhE1 | RhE | A | A | P | P | Y | V | F | C | S | L | S | P | R | | |
| 260 | RhE2 | RhE | T | V | D | L | S | P | A | F | L | F | F | G | R | L | A |
| 261 | RhE3 | RhE | S | L | A | P | Y | S | L | R | I | L | R | V | G | S | A |
| 262 | RhE4 | RhE | R | N | V | L | P | I | F | N | D | V | Y | W | I | A | L |
| 263 | RhE5 | RhE | R | N | V | P | P | I | F | N | D | V | Y | W | I | A | F |
| 264 | RhE6 | RhE | I | N | N | T | F | T | W | | | | | | | | |
| 265 | RhE7 | RhE | Y | E | L | N | L | M | T | | | | | | | | |
| 266 | RhE8 | RhE | W | G | I | T | V | E | T | A | Y | G | T | A | | | |
| 267 | RhE9 | RhE | N | Q | F | L | L | W | E | T | R | S | M | R | | | |
| 268 | RhE10 | RhE | N | V | S | L | T | N | L | T | Y | K | P | R | | | |
| 269 | RhE11 | RhE | Y | T | P | P | D | W | S | W | W | P | A | P | | | |
| 270 | RhE12 | RhE | I | K | N | T | S | L | Q | Q | L | V | T | A | | | |
| 271 | RhE13 | RhE | E | W | L | A | Y | D | G | I | R | A | Y | S | | | |
| 272 | RhE14 | RhE | H | A | K | V | Q | V | S | S | P | F | P | P | | | |
| 273 | RhE15 | RhE | L | S | S | Q | F | K | Q | | | | | | | | |
| 274 | RhE16 | RhE | G | T | F | K | W | Y | Q | | | | | | | | |
| 275 | RhE17 | RhE | T | K | M | D | Q | S | T | | | | | | | | |
| 276 | RhE18 | RhE | I | P | E | E | A | L | R | A | R | F | K | T | | | |
| 277 | RhE19 | RhE | S | F | Q | D | A | L | L | S | R | W | Y | S | | | |
| 278 | RhE20 | RhE | S | N | L | R | S | W | L | F | P | F | D | R | V | G | N |
| 279 | RhE21 | RhE | P | G | P | M | F | G | G | S | Y | I | P | S | L | H | H |
| 280 | RhE22 | RhE | Y | P | Q | G | L | W | R | | | | | | | | |
| 281 | RhE23 | RhE | Y | P | Q | G | E | W | R | | | | | | | | |
| 282 | RhE24 | RhE | G | P | S | Y | Y | A | L | I | T | R | Y | L | G | A | A |
| 283 | RhE25 | RhE | G | L | S | Y | Y | A | L | I | T | R | Y | L | G | A | A |
| 284 | RhE26 | RhE | W | P | P | I | S | R | H | | | | | | | | |
| 285 | RhE27 | RhE | A | H | S | W | L | P | G | A | G | L | L | M | | | |
| 286 | RhE28 | RhE | S | L | T | H | S | P | R | T | P | I | L | A | | | |
| 287 | RhE29 | RhE | F | P | N | L | R | E | R | H | E | P | L | F | | | |
| 288 | RhE30 | RhE | G | H | S | Y | H | A | L | I | T | R | Y | L | G | A | A |
| 289 | RhE31 | RhE | W | A | S | Y | Y | A | L | I | T | R | Y | L | G | A | A |
| 290 | RhE32 | RhE | D | F | A | Q | A | L | F | L | R | Y | V | V | S | G | L |
| 291 | RhE33 | RhE | H | R | W | M | P | H | V | F | A | V | R | Q | G | A | L |
| 292 | RhE34 | RhE | W | A | S | F | Y | A | S | S | Y | R | D | S | R | L | L |
| 293 | RhE35 | RhE | R | L | P | A | T | I | R | A | L | L | G | R | D | V | R |
| 294 | RhE36 | RhE | R | L | P | A | T | I | R | A | L | L | G | R | D | V | W |
| 295 | Rhe1 | Rhe | V | P | R | H | N | P | I | | | | | | | | |
| 296 | Rhe2 | Rhe | P | K | A | F | Q | Y | G | G | R | A | V | G | G | L | W |
| 297 | Rhe3 | Rhe | D | S | G | A | L | F | N | H | I | F | M | P | G | P | F |
| 298 | Rhe4 | Rhe | L | H | S | S | H | L | P | P | D | D | R | R | W | G | L |
| 299 | Rhe5 | Rhe | P | R | Q | F | P | L | R | D | L | Y | T | F | R | Y | W |
| 300 | Rhe6 | Rhe | G | A | A | N | L | Y | V | S | S | F | L | I | P | L | H |
| 301 | Rhe7 | Rhe | R | N | V | P | P | I | F | N | D | V | Y | W | I | A | L |
| 302 | Rhe8 | Rhe | Q | P | R | G | V | T | V | H | G | D | A | W | R | V | A |
| 303 | Rhe9 | Rhe | G | L | D | L | L | G | D | V | R | I | P | V | V | R | R |
| 304 | Rhe10 | Rhe | A | Y | W | D | L | Y | G | V | G | F | A | F | S | A | P |
| 305 | Rhe11 | Rhe | I | W | T | I | T | G | S | T | K | Q | A | F | D | R | S |
| 306 | Rhe12 | Rhe | E | D | W | F | S | A | S | I | R | R | V | P | T | F | A |
| 307 | Rhe13 | Rhe | A | V | K | L | R | P | S | S | C | W | L | K | S | T | C |
| 308 | Rhe14 | Rhe | R | F | D | G | I | D | L | R | V | S | F | V | S | R | P |
| 309 | Rhe15 | Rhe | A | S | L | H | P | V | P | K | T | W | F | S | L | L | S |
| 310 | Rhe16 | Rhe | L | A | P | P | P | S | P | | | | | | | | |
| 311 | Rhe17 | Rhe | A | V | T | F | N | S | Y | F | G | F | S | T | T | S | V |

Example 4

Specific binding of synthetic peptides to the original Duffy monoclonal antibodies

The relevance of the phage-peptides selected by biopanning was tested in immunological studies. Initially, 20 "Fy^{b}" phage (Peptides Fyb1-13, Fyb 20-21, Fyb 24-28; see Table 2 for sequence details) were selected for investigation by both phage ELISA and SPOTs analysis, to confirm that synthetic peptides could bind specifically the original monoclonal antibodies.

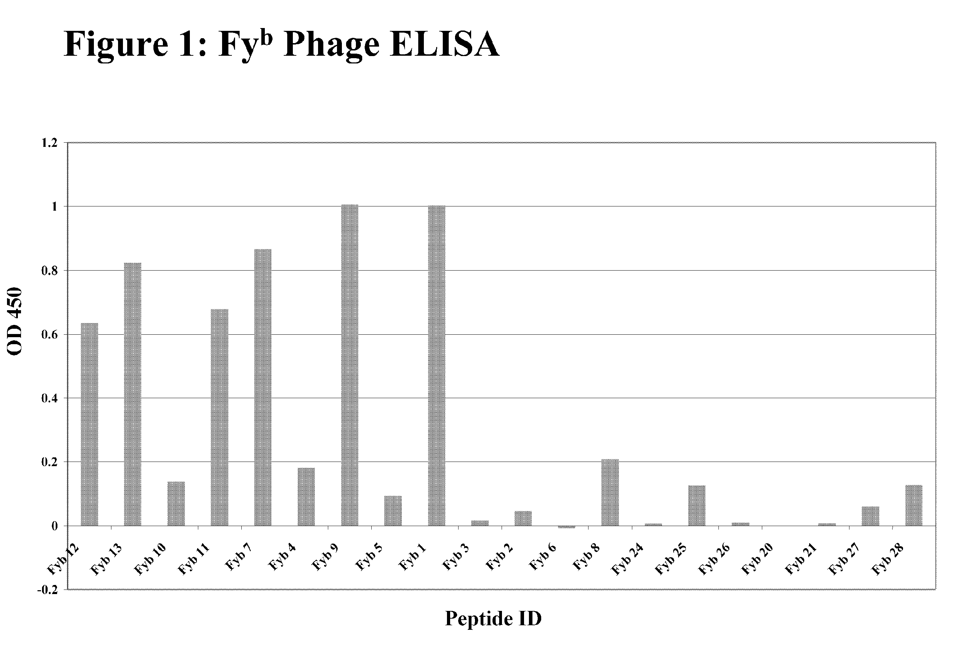

The phage ELISA results confirmed the binding of the anti- Fy^{b} monoclonal, LM447. A 10µg/ml stock of this monoclonal antibody was prepared in ELISA coating buffer and 150µl aliquots were added to wells of an Immulon 4 ELISA plate, and then incubated overnight at 4°C. Unbound antibody was discarded and the wells were blocked with 350µl of blocking buffer for 1 hour at 4°C. The wells were washed three times with 300µl TBS/0.5% Tween 20 and 100µl of selected phage culture supernatants were added to appropriate wells for 1 hour at room temperature. Unbound phage were removed and the wells washed six times with 300µl TBS/0.5% Tween 20. A 1:5000 dilution of horseradish peroxidase (HRP) conjugated anti-M13, secondary antibody was prepared in blocking buffer and 100µl aliquots added to each well. Following incubation for 1 hour at room temperature the wells were washed six times with 300µl TBS/0.5% Tween 20. To develop the reactions, 200µl aliquots of o-phenylenediamine dihydrochloride (OPD) substrate (Sigma) was added to each well. The absorbance at 450nm was measured following incubation at room temperature for 1 hour. The absorbance values at 450nm, confirmed the binding of 14 (Peptides Fyb1, Fyb2, Fyb4-5, Fyb7-13, Fyb25, 27 & 28) of the 20 phage assayed (see Figure 1).

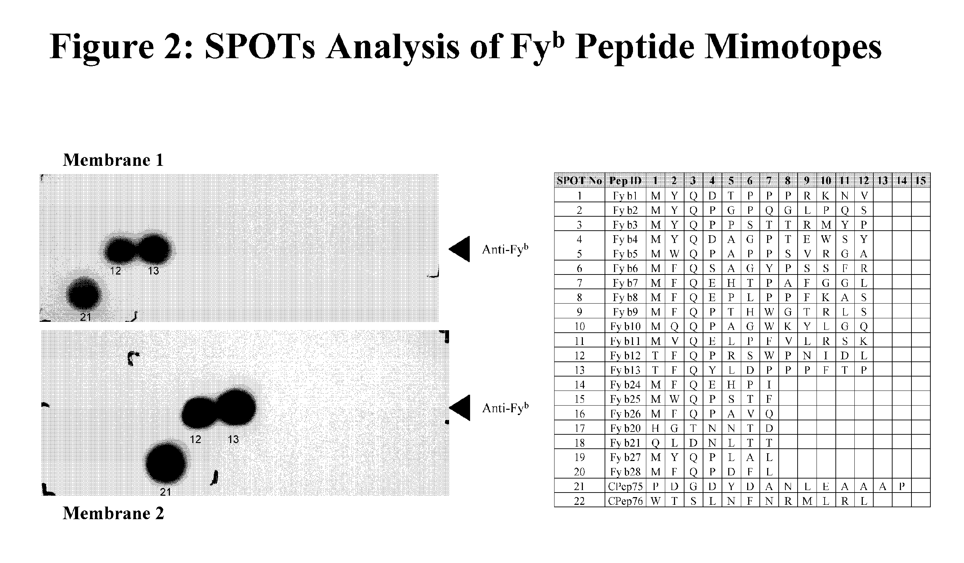

The 20 Fy^{b} peptide mimotopes were synthesised in duplicate, by SPOTS. Binding experiments using the anti-Fy^{b} antibody (LM447) showed that the antibody successfully bound 2 of the 20 peptide sequences (Peptides Fyb12 & Fyb13) tested (Figure 2). Binding was also observed in Spot 21 with the Peptide CPep75 (the positive control for the assay). Despite Peptides Fyb1, Fyb7, Fyb9 and Fyb11 producing phage ELISA signals equally as strong as Peptides Fyb12 and Fyb13, these 4 peptides failed to react with the anti-Fy^{b} antibody (LM447) when synthesised as SPOTS.

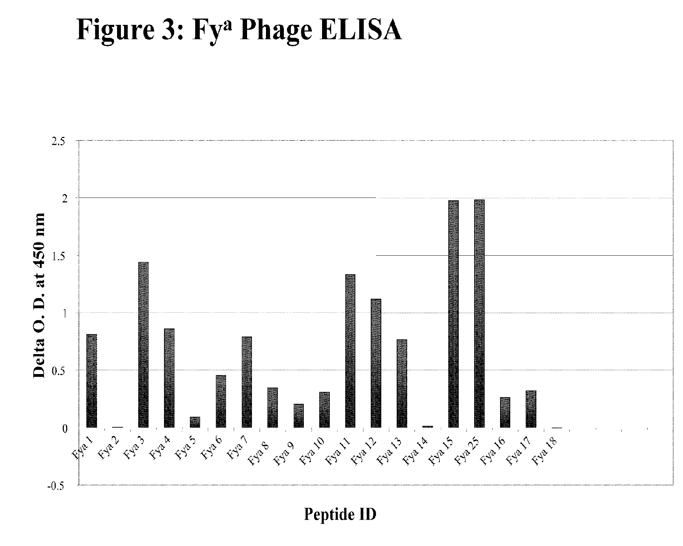

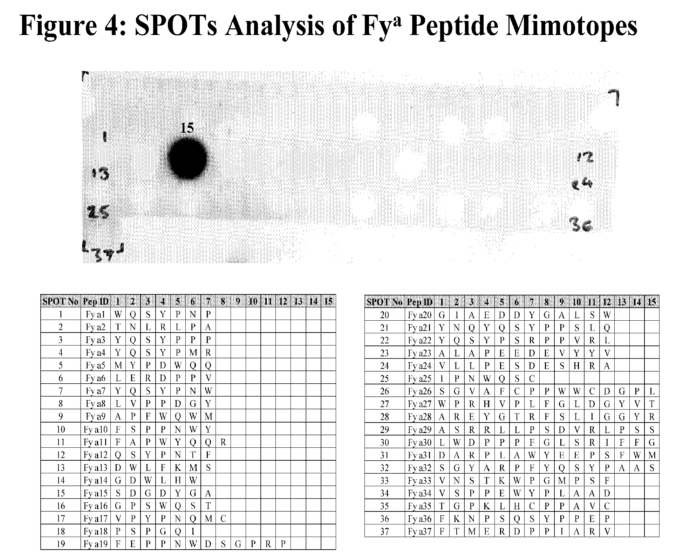

Phage ELISA analysis of 19 "Fy^{a}" phage-peptide clones (Peptides Fya1-18 & Fya25; see Table 3 for sequence details) confirmed the binding specificity of the LM487 antibody for the Fy^{a} phage-peptides. Figure 3 shows that only 3 of 19 (Peptides Fya2, Fya14 & Fya18) failed to bind the antibody. These 19 peptides were amongst 37 (Peptides Fya1-37) synthesised as SPOTs and a strong positive signal was only observed with Peptide Fya15 (Spot 15), following hybridisation of the membrane with the anti-Fy^{a} antibody (Figure 4).

These results mirrored the observations obtained for the Fy^{b} phage-peptides when tested in parallel by both phage ELISA and SPOTS. A positive phage ELISA signal did not automatically guarantee a positive signal when the corresponding peptide mimotope was synthesised and tested by SPOTs hybridisation. Moreover, sequence analysis identified no obvious sequence similarities amongst either positive or negative peptides.

The results from both the Fy^{a} and Fy^{b} phage ELISA and SPOTs assays suggested that the mode of presentation of the peptide is important in antibody binding. Peptides that form part of a fusion protein in the phage coat may adopt a different conformation when synthesised on a membrane by SPOTS. Without the surrounding protein, antibody recognition may be subsequently affected. Therefore, rather than simply using ELISA to select phage-peptide clones for analysis by SPOTS, all phage-peptides (Fy^{b}, Fy^{a}, RhD and RhEe) selected by biopanning were characterised by DNA sequence analysis, prior to SPOTs analysis with the monoclonal antibody originally used in their selection, in order to ensure no potentially diagnostic peptide was overlooked.

Reciprocal Hybridisation to Identify Cross-reactive Peptides

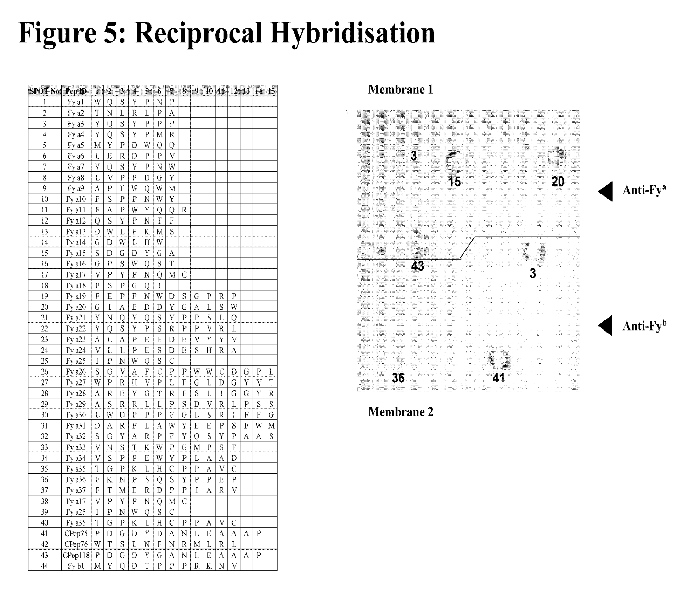

A series of 44 peptides was synthesised in duplicate (Figure 5). Spots 1-37 were Fya peptide mimotopes (Fya 1-37), Spots 38-40 were repeats of Peptides Fya17, Fya25 and Fya35, respectively and Spots 41-44 were Control peptides (Spot 41 was the immunisation peptide [Cpep75] used to generate the anti-Fy^{b} antibody; Spot 42 an irrelevant 12-mer [Cpep76]; Spot 43 the immunisation peptide [CPep118] used to generate the anti-Fy^{a} antibody and Spot 44 an Fyb peptide mimotope [Fyb1]). Hybridisation of the membranes, in parallel, with the anti- Fy^{a} and anti-Fy^{b} monoclonal antibodies, confirmed the Fy^{a}-specificity of a number of the peptides (Peptide spots 15, 20, and 43) but also identified peptides capable of binding the anti-Fy^{b} antibody (Spots 36 and 41) and a further peptide displaying cross-reactivity with both antibodies (Spot 3). The signals observed with Spots 43 and 41 were not unexpected as these 2 peptides were included as positive controls for the anti- Fy^{a} and anti-Fy^{b} monoclonal antibodies, respectively.

Generation of consensus sequence-derived synthetic Fyb peptide mimotopes by amino acid substitution

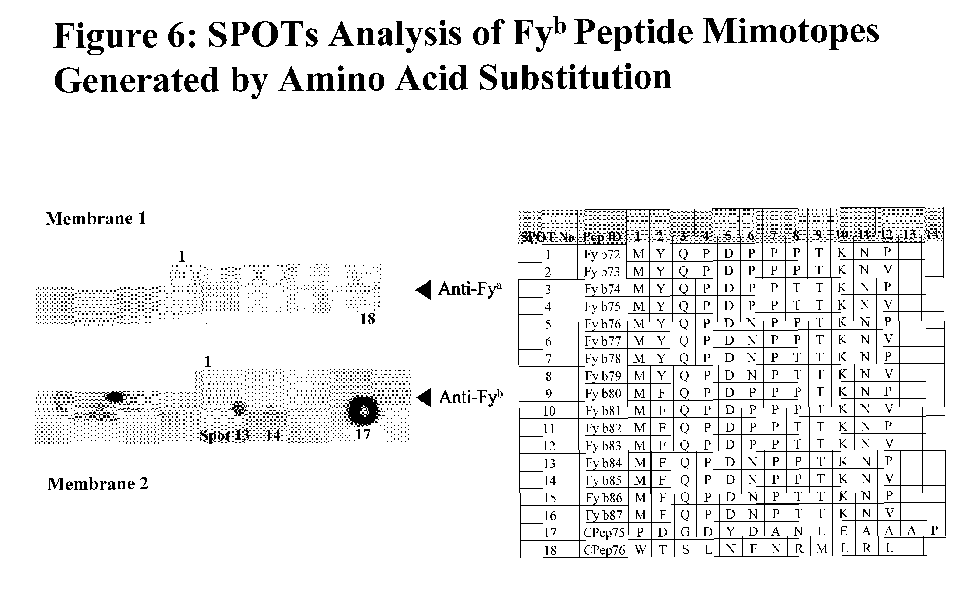

Sequence analysis of the 100 unique "Fyb" peptide mimotopes (Fyb1-71 & Fyb88-116) had identified 2 consensus **sequences. Consensus 1** : M(F/Y)QPD(N/P)(P/L)(T/P)T(K/L)(N/Q)(P/V/A/S) contains a series of positions (amino acid 2, 6, 7, 8, 10, 11 & 12) which have a limited number of residues occurring with equal frequency. A series of 16 peptide mimotopes (Peptides Fyb72-87; see Table 2) were synthesised by SPOTs, with amino acid substitutions at positions 2, 6, 8 and 12. On testing, 2 of these consensus sequence-derived peptide mimotopes (Peptide Fyb84; Spot 13 and Peptide Fyb85; Spot 14) proved capable of binding the anti-Fy^{b} antibody LM447 (see Figure 6). Analysis of these 2 synthetic, peptide mimotope sequences identified a single amino acid difference at position 12. The strength of signal observed for these 2 sequences was however dramatically lower than that achieved with Peptide CPep75 (Spot 17), the positive control used for this assay. This result shows that peptide mimotopes may also be artificially generated through amino acid substitution experiments and not simply through biopanning.

Example 5

Specific binding of antibodies to synthetic Rh peptides determined by SPOTs

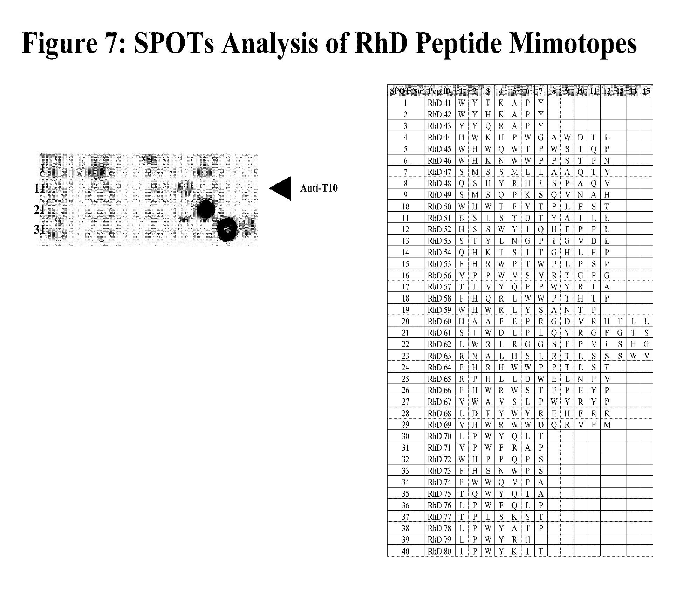

The RhD, RhE and Rhe peptide mimotopes (see Tables 4 & 5) were synthesised by SPOTs and hybridisation of the membranes was undertaken with the appropriate antibodies (Table 1). Figures 7-10 show examples of peptide mimotopes reacting specifically with the antibody originally used in their selection.

Figure 7 shows the results following hybridisation of a subset of the anti-RhD antibody-selected peptide mimotopes with the monoclonal antibody T10. 40 peptides (RhD41-RhD80) were synthesised and positive signals were observed in 11 spots (Spots 1-3, 8, 17, 27, 28, 31, 37, 39 & 40; see Table for peptide sequences).

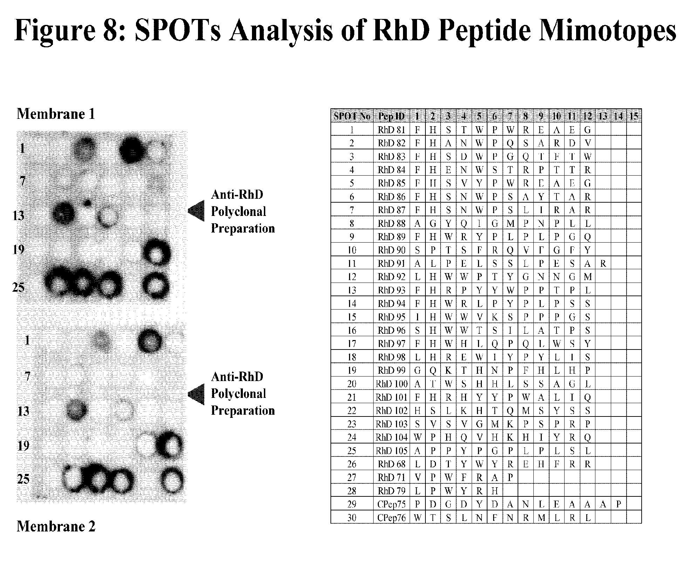

A further 28 RhD peptide mimotopes were synthesised by SPOTS. Peptide mimotopes RhD81-105 (Spots 1-25) were originally selected with the polyclonal anti-RhD preparation and Peptides RhD68, RhD71 and RhD79 (Spots 26-28) were identified through biopanning with the anti-RhD antibody T10. The peptides CPep75 (Spot 29) and CPep76 (Spot 30) were included in the assay as negative controls. Figure 8 identifies a series of 11 peptides recognised by the polyclonal anti-RhD (Spots 3, 5, 6, 12, 14, 16, 24, 26-28 & 30; see Table for peptide sequences). This series of peptides includes 3 peptides (Spots 26-28) originally selected with T10. This result strongly suggested that peptides identified with individual monoclonal antibodies (eg T10) mimic epitopes of the antigen to which the polyclonal antibody was raised.

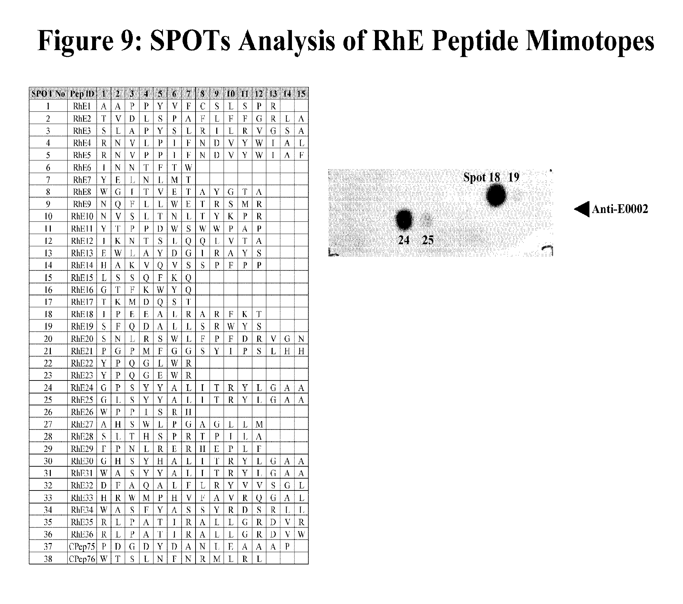

Figure 9 shows the results following hybridisation of the 36 RhE peptide mimotopes with the anti-RhE monoclonal antibody E0002. The peptides CPep75 (Spot 37) and CPep76 (Spot 38) were included in the assay as negative controls. Specific binding of the antibody was achieved with 4 spots (Spot 18: Peptide mimotope RhE18; Spot 19: RhE19; Spot 24: RhE24 & Spot 25: RhE25).

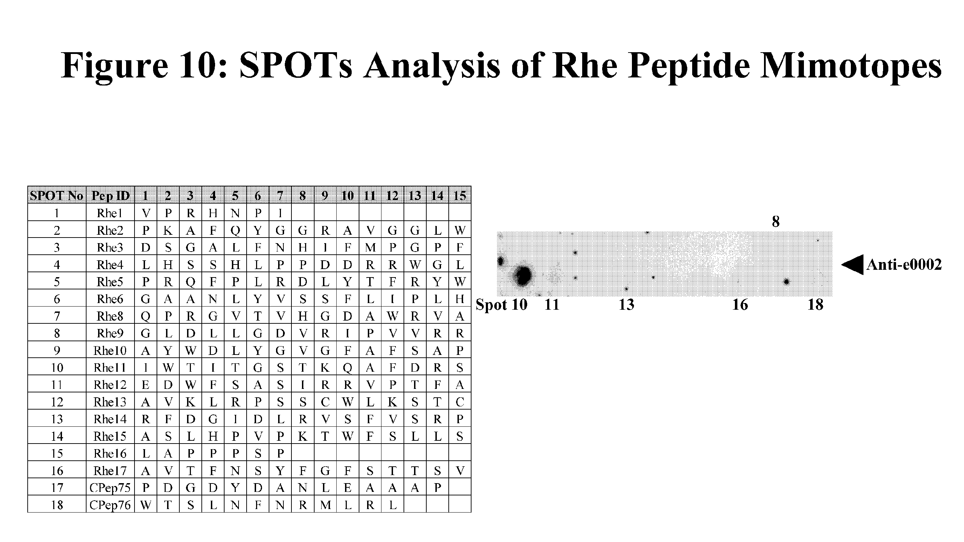

Figure 10 shows the results following hybridisation of the 16 Rhe peptide mimotopes with the anti-Rhe monoclonal antibody e0002. The peptides CPep75 (Spot 17) and CPep76 (Spot 18) were included in the assay as negative controls. Strong positive signals were observed with 2 spots (Spots 10 & 13; see Table for peptide sequences). A further 3 weakly, positive spots were also noted (Spots 8, 11 & 16) however these signals were only as strong as that achieved with the control peptide CPep 76 (Spot 18) which was included in the assay as a negative control.

Overall, the results achieved with the Rh peptide mimotopes were comparable to those obtained with the Fya and Fyb peptides and Fy^{a} and Fy^{b} monoclonal antibodies. SPOTs analysis identified Rh peptides that reacted specifically with their cognitive antibody.

Example 6

Specific binding of antibodies to synthetic peptide mimotopes bound to microsphere beads

Further confirmation that the peptide mimotopes can specifically bind antibodies raised to native blood group antigens was achieved following the immobilisation of peptide mimotopes onto polystyrene LiquiChip Ni-NTA microsphere beads (Qiagen).

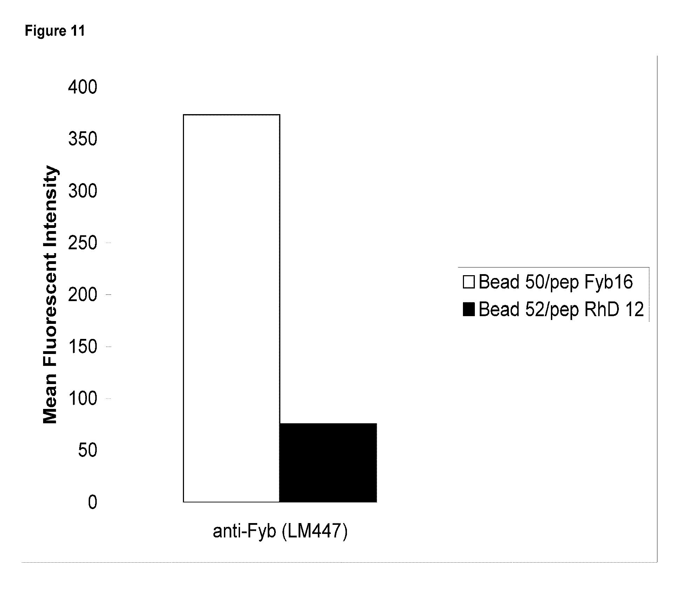

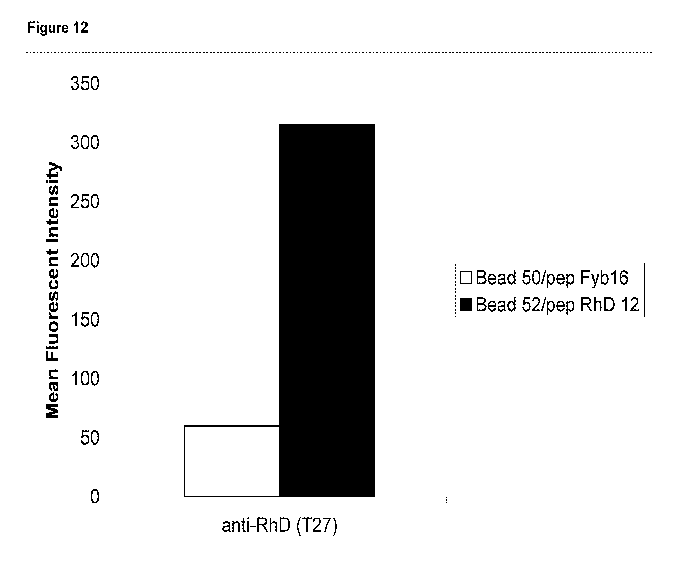

Peptide mimotopes (e.g. Fyb16; RhD12) were synthesised with a C-terminal tag consisting of 6 Histidine amino acids. Stock solutions (4µg/ml) of the 6xHis tagged peptide mimotopes were prepared in PBS/0.1%BSA. The peptides were immobilised onto the beads using the Ni-NTA groups attached to the surface of the beads, as follows. A 50µl volume of a 4µg/ml peptide stock (Fyb16 or RhD12) was mixed with an equal volume of LiquiChip Ni-NTA beads (Bead 50 or Bead 52, respectively). Following incubation with mixing at 4°C overnight, the Bead/peptide mixture was diluted with 900µl of PBS/0.1%BSA. The Bead/peptide mixture was vortexed for 2 minutes and then a 10µl volume was aliquoted into the well of a 96-well, round-bottomed ELISA plate (Nunc). A 10µl volume of the test monoclonal antibody (LM447: anti-Fy^{b} or T27: anti-RhD) was added to the well of the ELISA plate and the reaction volume was made up to 60µl with PBS/1%BSA. The plate was incubated at room temperature for 90 minutes, in the dark and then a 10µl volume (200ng) of phycoerythrin conjugated sheep anti-mouse IgG (added to wells containing the LM447: anti-Fy^{b} monoclonal antibody) or phycoerythrin conjugated goat anti-human IgM (added to wells containing the T27: anti-RhD monoclonal antibody) was added into the well. The plate was incubated at room temperature for a further 90 minutes, in the dark. The plate was briefly vortexed, then loaded into a Luminex 100 Analyser (Luminex). Confirmation of bound monoclonal antibody was determined by the detection of a phycoerythrin fluorochrome signal. The intensity of the signal equates to the quantity of monoclonal antibody bound to the immobilised peptide mimotope. The results are set out in Figures 11 and 12 where specific binding of anti-Fyb monoclonal antibody (LM447) to Peptide Mimotope Fyb16 and specific binding of the anti-RhD monoclonal antibody (T27) to Peptide Mimotope RhD12 is shown.

Example 7

Specific binding of antibodies to synthetic peptide mimotopes bound to superparamagnetic beads

The ability of peptide mimotopes to specifically bind antibodies was further tested using superparamagnetic polystyrene beads (TALON Dynabeads; Invitrogen) in a modified gel agglutination assay.

A 50µl volume of the magnetic beads was added to 700µl of TBS (50mM Tris-HCl pH7.5; 150mM NaCl). Following mixing for 5 minutes at room temperature, the magnetic beads were isolated using a Magnetic Particle Concentrator (Invitrogen). The supernatant was discarded and the beads were washed for 5 minutes in 700 µl of TBS. The beads were again separated from the buffer using the MPC magnet, and then resuspended in 700 µl of TBS. Peptide mimotopes (e.g. Fyb16; RhD12) were synthesised with a C-terminal tag consisting of 6 Histidine amino acids. Stock solutions (100µg/ml) of the 6xHis tagged peptide mimotopes were prepared in PBS.

A 50µl volume of diluted TALON beads was placed in the reaction chamber of an NaCl gel card (ID Microtyping System; Diamed), together with a 50µl volume of diluted His-tagged peptide mimotope (e.g. RhD12) and 100µl of test human monoclonal antibody (e.g. T27: anti-RhD). The gel card was incubated at 37°C for 1 hour in an Incubator-ID (DiaMed). Following incubation, the card was centrifuged for 10 minutes in an ID Centrifuge 24S (DiaMed). Antibody-positive reactions are visualised by a layer of beads upon the surface of the gel matrix. In negative reactions, non-agglutinated beads pass through the gel matrix and form a pellet.

To test the binding of a murine monoclonal antibody (e.g. LM447: anti-Fy^{b} monoclonal antibody), a 1µl volume of sheep anti-mouse IgG is first added to the reaction chamber of an NaCl gel card and allowed to equilibrate with the gel matrix for 10 minutes at room temperature, prior to the addition of a 50µl volume of diluted TALON beads, a 50µl volume of diluted His-tagged peptide mimotope (e.g. Fyb16) and 100µl of the test murine monoclonal antibody (e.g. LM447: anti-Fy^{b}) . The gel card was incubated at 37°C for 1 hour in an ID Incubator (DiaMed). Following incubation, the card was centrifuged for 10 minutes in an ID centrifuge (DiaMed). Again the reaction was read visually.

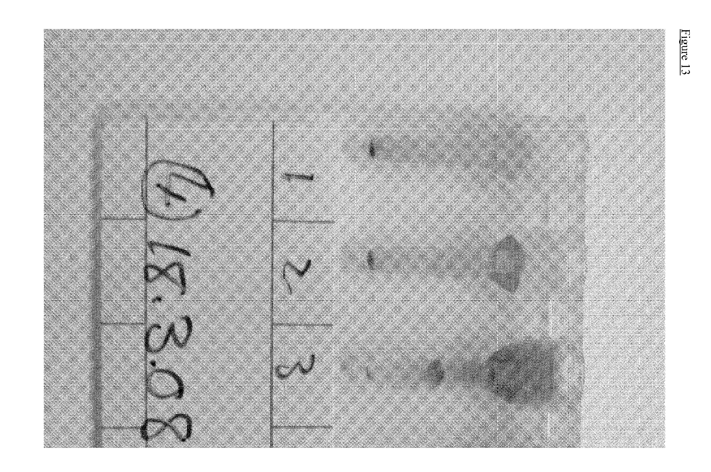

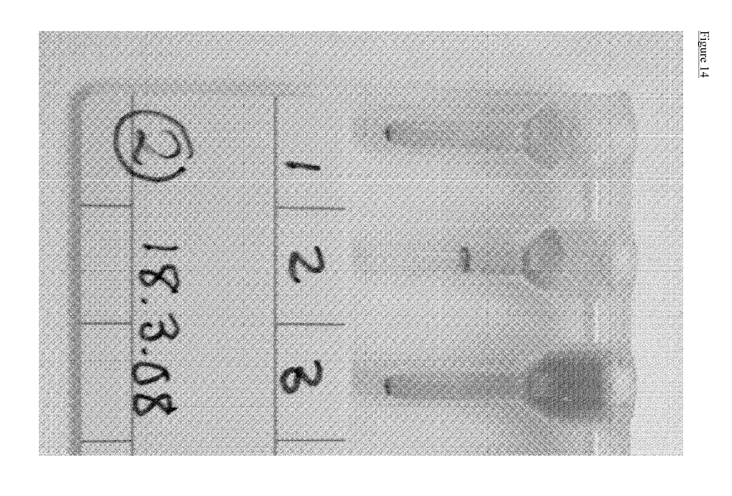

These examples demonstrate that the synthetic peptide mimotopes are capable of specifically binding antibodies raised to native blood group antigens even when bound to a solid support. In these examples the solid support used is polystyrene Ni-NTA microsphere beads or supermagnetic polystyrene beads but it is envisaged that the synthetic peptide mimotopes will be capable of specifically binding antibodies raised to native blood group antigens when immobilised on any suitable support.
The results are identified in Figures 13 and 14 whereby specific binding of anti-RhD monoclonal antibody (T27) to Peptide Mimotope RhD12 and specific binding of the anti-Fy^{b} monocolonal antibody (LM447) to Peptide Mimotope Fyb16 is shown.
Aspects of the present invention include:
1. A mimotope selected from at least one of SEQ ID Nos 1 to 311 or a peptide fragment, an immunoreactive analogue or derivative or a cross-reactive sequence thereof.
2. A method for detecting an antibody to a blood group antigen in a sample, the method comprising:
   a) reacting a mimotope of said blood group antigen with the sample to be tested,
   b) detecting any reaction between the sample and the mimotope,
   c) wherein a reaction between the sample and the mimotope is indicative of the presence of an antibody to the blood group antigen.
3. A method according to aspect 2 wherein before step a) the mimotope is immobilised on a solid support.
4. A method according to aspect 3 wherein the solid support is selected from a membrane, glass slide or bead.
5. A method according to any of aspects 2 to 4 wherein the antibody is an alloantibody.
6. A method of diagnosing diseases or a reaction to blood products involving the generation of alloantibodies or autoantibodies, the method comprising using a mimotope of a blood group antigen to identify antibodies associated with the disease.
7. A method according to aspect 6 wherein the disease is HTR, haemolytic disease of the newborn or autoimmune haemolytic anaemia.
8. A method of diagnosing according to either aspects 6 or 7 wherein the method of detecting is according to any of aspects 2 to 5.
9. A method according to any preceding aspect wherein the blood group antigen is selected from Rhesus, Kell, Duffy, Kidd, ABO, MNS, P, Lutheran, Lewis, Diego, Yt, Xg, Scianna, Dombrock, Colton, Landsteiner-Wiener, Chido-Rodgers, Hh, Kx, Gerbich, Cromer, Knops, Indian, Ok, Raph, JMH, I, Globoside or GIL antigen mimotopes.
10. A device for detecting the presence of an antibody to a blood group antigen in a sample, the device comprising an array of mimotopes of a blood group antigen.
11. A device according to aspect 10 wherein the mimotope is immobilised on a solid support.

## Claims

1. A method for detecting an antibody to a blood group antigen in a sample, the method comprising:
a)reacting a mimotope of said blood group antigen with the sample to be tested,
b) detecting any reaction between the sample and the mimotope,
c) wherein a reaction between the sample and the mimotope is indicative of the presence of an antibody to the blood group antigen.

2. A method according to claim 1 wherein before step a) the mimotope is immobilised on a solid support.

3. A method according to claim 2 wherein the solid support is selected from a membrane, glass slide or bead.

4. A method according to any of claims 1 to 3 wherein the antibody is an alloantibody.

5. A method of diagnosing diseases or a reaction to blood products involving the generation of alloantibodies or autoantibodies, the method comprising using a mimotope of a blood group antigen to identify antibodies associated with the disease.

6. A method according to claim 5 wherein the disease is haemolytic transfusion reaction, haemolytic disease of the newborn or autoimmune haemolytic anaemia.

7. A method of diagnosing according to either claim 5 or 6 wherein the method of detecting is according to any of claims 1 to 4.

8. A method according to any preceding claim wherein the blood group antigen is selected from Rhesus, Kell, Duffy, Kidd, ABO, MNS, P, Lutheran, Lewis, Diego, Yt, Xg, Scianna, Dombrock, Colton, Landsteiner-Wiener, Chido-Rodgers, Hh, Kx, Gerbich, Cromer, Knops, Indian, Ok, Raph, JMH, I, Globoside or GIL antigen mimotopes.

9. A method according to any preceding claim, wherein the mimotope comprises a sequence selected from at least one of SEQ ID No 161, SEQ ID Nos 1 to 160 or SEQ ID Nos 162 to 311, or a peptide fragment, an immunoreactive analogue or derivative or a cross-reactive sequence thereof.

10. A device for detecting the presence of an antibody to a blood group antigen in a sample, the device comprising an array of mimotopes of a blood group antigen.

11. A device according to claim 10 wherein the mimotope array comprises one or more of the amino acid sequences of SEQ ID No 161, SEQ ID Nos 1 to 160 or SEQ ID Nos 162 to 311, or a peptide fragment, an immunoreactive analogue or derivative or a cross-reactive sequence thereof.

12. A device according to either claim 10 or 11 wherein the mimotope is immobilised on a solid support.

13. A mimotope selected from at least one of SEQ ID No 161, SEQ ID Nos 1 to 160 or SEQ ID Nos 162 to 311, or a peptide fragment, an immunoreactive analogue or derivative or a cross-reactive sequence thereof.


SEQUENCE LISTING

<110>  The Common Services Agency for the Scottish Health Service

<120>  DIAGNOSTIC ASSAY

<130>  B1576

<150>  GB0706558.4
<151>  2007-04-03

<160>  311

<170>  PatentIn version 3.2

<210>  1
<211>  12
<212>  PRT
<213>  Artificial

<220>
<223>  Synthetic peptide

<400>  1

Met Tyr Gln Asp Thr Pro Pro Pro Arg Lys Asn Val
1               5                   10


<210>  2
<211>  12
<212>  PRT
<213>  Artificial

<220>
<223>  Synthetic peptide

<400>  2

Met Tyr Gln Pro Gly Pro Gln Gly Leu Pro Gln Ser
1               5                   10


<210>  3
<211>  12
<212>  PRT
<213>  Artificial

<220>
<223>  Synthetic peptide

<400>  3

Met Tyr Gln Pro Pro Ser Thr Thr Arg Met Tyr Pro
1               5                   10


<210>  4
<211>  12
<212>  PRT
<213>  Artificial

<220>
<223>  Synthetic peptide

<400>  4

Met Tyr Gln Asp Ala Gly Pro Thr Glu Trp Ser Tyr

1                   5                        10


<210>  5
<211>  12
<212>  PRT
<213>  Artificial

<220>
<223>  Synthetic peptide

<400>  5

Met Trp Gln Pro Ala Pro Pro Ser Val Arg Gly Ala
1                   5                        10


<210>  6
<211>  12
<212>  PRT
<213>  Artificial

<220>
<223>  Synthetic peptide

<400>  6

Met Phe Gln Ser Ala Gly Tyr Pro Ser Ser Phe Arg
1                   5                        10


<210>  7
<211>  12
<212>  PRT
<213>  Artificial

<220>
<223>  Synthetic peptide

<400>  7

Met Phe Gln Glu His Thr Pro Ala Phe Gly Gly Leu
1                   5                        10


<210>  8
<211>  12
<212>  PRT
<213>  Artificial

<220>
<223>  Synthetic peptide

<400>  8

Met Phe Gln Glu Pro Leu Pro Pro Phe Lys Ala Ser
1                   5                        10


<210>  9
<211>  12
<212>  PRT
<213>  Artificial

<220>
<223>  Synthetic peptide

<400>  9

Met Phe Gln Pro Thr His Trp Gly Thr Arg Leu Ser
1               5                   10

<210>  10
<211>  12
<212>  PRT
<213>  Artificial

<220>
<223>  Synthetic peptide

<400>  10

Met Gln Gln Pro Ala Gly Trp Lys Tyr Leu Gly Gln
1               5                   10

<210>  11
<211>  12
<212>  PRT
<213>  Artificial

<220>
<223>  Synthetic peptide

<400>  11

Met Val Gln Glu Leu Pro Phe Val Leu Arg Ser Lys
1               5                   10

<210>  12
<211>  12
<212>  PRT
<213>  Artificial

<220>
<223>  Synthetic peptide

<400>  12

Thr Phe Gln Pro Arg Ser Trp Pro Asn Ile Asp Leu
1               5                   10

<210>  13
<211>  12
<212>  PRT
<213>  Artificial

<220>
<223>  Synthetic peptide

<400>  13

Thr Phe Gln Tyr Leu Asp Pro Pro Pro Phe Thr Pro
1               5                   10

<210>  14
<211>  12
<212>  PRT
<213>  Artificial

<220>
<223>  Synthetic peptide

<400>  14

Thr Tyr Gln His Asn Asn Pro Asp Ala Phe Arg Leu
1               5                   10


<210>  15
<211>  12
<212>  PRT
<213>  Artificial

<220>
<223>  Synthetic peptide

<400>  15

Met Phe Gln Ser Asn Thr Tyr Pro Ile Trp Asn Val
1               5                   10


<210>  16
<211>  12
<212>  PRT
<213>  Artificial

<220>
<223>  Synthetic peptide

<400>  16

Phe Gln Pro Asn Pro Pro Pro Ser Phe Ala Ala Pro
1               5                   10


<210>  17
<211>  12
<212>  PRT
<213>  Artificial

<220>
<223>  Synthetic peptide

<400>  17

Met Tyr Gln Gly Arg Asn Asp Pro His Leu Leu Pro
1               5                   10


<210>  18
<211>  12
<212>  PRT
<213>  Artificial

<220>
<223>  Synthetic peptide

<400>  18

Met Tyr Gln Pro Arg Asp Trp Arg Ser Ala Pro Pro
1               5                   10


<210>  19
<211>  12
<212>  PRT
<213>  Artificial

<220>
<223>  Synthetic peptide

<400>  19

Met Phe Asn Ala Asp Pro Thr Ile Pro Ala Gln Pro
1               5                   10


<210>  20
<211>  7
<212>  PRT
<213>  Artificial

<220>
<223>  Synthetic peptide

<400>  20

His Gly Thr Asn Asn Thr Asp
1               5


<210>  21
<211>  7
<212>  PRT
<213>  Artificial

<220>
<223>  Synthetic peptide

<400>  21

Gln Leu Asp Asn Leu Thr Thr
1               5


<210>  22
<211>  7
<212>  PRT
<213>  Artificial

<220>
<223>  Synthetic peptide

<400>  22

His Tyr Tyr His Asn Thr Asp
1               5


<210>  23
<211>  7
<212>  PRT
<213>  Artificial

<220>
<223>  Synthetic peptide

<400>  23

His Ala Leu Pro Leu His Ala
1               5


<210>  24
<211>  7
<212>  PRT

<213>  Artificial

<220>
<223>  Synthetic peptide

<400>  24

Met Phe Gln Glu His Pro Ile
1               5


<210>  25
<211>  7
<212>  PRT
<213>  Artificial

<220>
<223>  Synthetic peptide

<400>  25

Met Trp Gln Pro Ser Thr Phe
1               5


<210>  26
<211>  7
<212>  PRT
<213>  Artificial

<220>
<223>  Synthetic peptide

<400>  26

Met Phe Gln Pro Ala Val Gln
1               5


<210>  27
<211>  7
<212>  PRT
<213>  Artificial

<220>
<223>  Synthetic peptide

<400>  27

Met Tyr Gln Pro Leu Ala Leu
1               5


<210>  28
<211>  7
<212>  PRT
<213>  Artificial

<220>
<223>  Synthetic peptide

<400>  28

Met Phe Gln Pro Asp Phe Leu
1               5


<210>  29

<211>  7
<212>  PRT
<213>  Artificial

<220>
<223>  Synthetic peptide

<400>  29

Met Phe Gln Glu Leu Val Ile
1               5


<210>  30
<211>  7
<212>  PRT
<213>  Artificial

<220>
<223>  Synthetic peptide

<400>  30

Met Phe Gln Ala Asn Thr Val
1               5


<210>  31
<211>  7
<212>  PRT
<213>  Artificial

<220>
<223>  Synthetic peptide

<400>  31

Met Phe Gln Pro Leu His Arg
1               5


<210>  32
<211>  7
<212>  PRT
<213>  Artificial

<220>
<223>  Synthetic peptide

<400>  32

Met Phe Gln Pro Thr Asp His
1               5


<210>  33
<211>  7
<212>  PRT
<213>  Artificial

<220>
<223>  Synthetic peptide

<400>  33

Met Phe Gln Pro Leu Pro Ser
1               5

<210>  34
<211>  7
<212>  PRT
<213>  Artificial

<220>
<223>  Synthetic peptide

<400>  34

Met Phe Gln Pro Gln Tyr His
1               5


<210>  35
<211>  7
<212>  PRT
<213>  Artificial

<220>
<223>  Synthetic peptide

<400>  35

Met Phe Gln Pro Pro Leu Lys
1               5


<210>  36
<211>  7
<212>  PRT
<213>  Artificial

<220>
<223>  Synthetic peptide

<400>  36

Met Tyr Gln Pro Pro Gly Ser
1               5


<210>  37
<211>  7
<212>  PRT
<213>  Artificial

<220>
<223>  Synthetic peptide

<400>  37

Met Tyr Gln Pro Arg Thr Leu
1               5


<210>  38
<211>  7
<212>  PRT
<213>  Artificial

<220>
<223>  Synthetic peptide

<400>  38

Met Trp Gln Pro Asp Ala Arg

1                   5


<210>  39
<211>  7
<212>  PRT
<213>  Artificial

<220>
<223>  Synthetic peptide

<400>  39

Met Tyr Gln Asp Leu Pro Tyr
1                   5


<210>  40
<211>  12
<212>  PRT
<213>  Artificial

<220>
<223>  Synthetic peptide

<400>  40

Met Trp Gln Pro Gln Pro Met Pro Gly Met Val Gly
1                   5                   10


<210>  41
<211>  12
<212>  PRT
<213>  Artificial

<220>
<223>  Synthetic peptide

<400>  41

Met Tyr Gln Pro Arg Asn Trp Ser Asp Ala Ser Gly
1                   5                   10


<210>  42
<211>  12
<212>  PRT
<213>  Artificial

<220>
<223>  Synthetic peptide

<400>  42

Thr Tyr Gln Pro Lys Ser Pro Ser Asn Phe Ala Asn
1                   5                   10


<210>  43
<211>  12
<212>  PRT
<213>  Artificial

<220>
<223>  Synthetic peptide

<400>  43

Met Phe Gln Pro Asn Asn Pro Pro Gly Ile His Ala
1               5                   10

<210>  44
<211>  7
<212>  PRT
<213>  Artificial

<220>
<223>  Synthetic peptide

<400>  44

Met Phe Gln Pro Tyr His Pro
1               5

<210>  45
<211>  7
<212>  PRT
<213>  Artificial

<220>
<223>  Synthetic peptide

<400>  45

Met Tyr Asn Pro Pro Trp Gly
1               5

<210>  46
<211>  7
<212>  PRT
<213>  Artificial

<220>
<223>  Synthetic peptide

<400>  46

Thr Tyr Gln Pro Leu Thr Trp
1               5

<210>  47
<211>  12
<212>  PRT
<213>  Artificial

<220>
<223>  Synthetic peptide

<400>  47

Val Ser His Thr Asn Asn Thr Asp Leu Arg Pro Pro
1               5                   10

<210>  48
<211>  12
<212>  PRT
<213>  Artificial

<220>
<223>  Synthetic peptide

<400>  48

Asp His Pro Pro Thr Leu Asn Arg Pro Pro Asn Val
1               5                   10


<210>  49
<211>  7
<212>  PRT
<213>  Artificial

<220>
<223>  Synthetic peptide

<400>  49

His Gly Val Ser Asn Thr Asp
1               5


<210>  50
<211>  7
<212>  PRT
<213>  Artificial

<220>
<223>  Synthetic peptide

<400>  50

Met Trp Gln Pro Arg Thr Met
1               5


<210>  51
<211>  7
<212>  PRT
<213>  Artificial

<220>
<223>  Synthetic peptide

<400>  51

His Tyr His Ser Asn Thr Asp
1               5


<210>  52
<211>  7
<212>  PRT
<213>  Artificial

<220>
<223>  Synthetic peptide

<400>  52

His Tyr Ala Ser Asn Thr Asp
1               5


<210>  53
<211>  7
<212>  PRT
<213>  Artificial

<220>
<223>  Synthetic peptide

<400>  53

Met Tyr Gln Pro Met Thr Ala
1               5


<210>  54
<211>  12
<212>  PRT
<213>  Artificial

<220>
<223>  Synthetic peptide

<400>  54

Met Tyr Gln Pro Asn Ser Thr Thr Gln Leu Tyr Asn
1               5                   10


<210>  55
<211>  12
<212>  PRT
<213>  Artificial

<220>
<223>  Synthetic peptide

<400>  55

Asn Phe Gln Pro Asn Asp Pro Pro Ala Arg Pro Ser
1               5                   10


<210>  56
<211>  12
<212>  PRT
<213>  Artificial

<220>
<223>  Synthetic peptide

<400>  56

Thr Tyr Gln His Met Pro Pro Pro His Ser Pro Phe
1               5                   10


<210>  57
<211>  12
<212>  PRT
<213>  Artificial

<220>
<223>  Synthetic peptide

<400>  57

Met Tyr Gln Glu Ser Ala Pro Pro Arg Gln Val Phe
1               5                   10


<210>  58
<211>  12
<212>  PRT

<213>  Artificial

<220>
<223>  Synthetic peptide

<400>  58

Met Trp Gln Pro Asn Ser Pro Ser Val Pro Pro Pro
1               5                   10


<210>  59
<211>  7
<212>  PRT
<213>  Artificial

<220>
<223>  Synthetic peptide

<400>  59

Ile Phe Asn Asp Pro Trp Met
1               5


<210>  60
<211>  7
<212>  PRT
<213>  Artificial

<220>
<223>  Synthetic peptide

<400>  60

Met Phe Gln Pro Leu Pro Ala
1               5


<210>  61
<211>  7
<212>  PRT
<213>  Artificial

<220>
<223>  Synthetic peptide

<400>  61

Met Tyr Gln Pro Thr His Trp
1               5


<210>  62
<211>  12
<212>  PRT
<213>  Artificial

<220>
<223>  Synthetic peptide

<400>  62

Met Phe Gln Pro Thr Leu Val Ala Pro Leu Gln Ala
1               5                   10


<210>  63

<211>  12
<212>  PRT
<213>  Artificial

<220>
<223>  Synthetic peptide

<400>  63

Met Phe Gln Ser Ser Pro Pro Pro Leu Gly His Ala
1               5                   10


<210>  64
<211>  12
<212>  PRT
<213>  Artificial

<220>
<223>  Synthetic peptide

<400>  64

Thr Phe Gln Pro Arg Leu Phe Ser Ser Trp Val His
1               5                   10


<210>  65
<211>  12
<212>  PRT
<213>  Artificial

<220>
<223>  Synthetic peptide

<400>  65

Met Phe Gln Ser Arg Gly Pro Asn Gln Leu Phe Pro
1               5                   10


<210>  66
<211>  12
<212>  PRT
<213>  Artificial

<220>
<223>  Synthetic peptide

<400>  66

Met Phe Gln Pro Ala Asn Pro Ser Gln Ser Thr Ala
1               5                   10


<210>  67
<211>  7
<212>  PRT
<213>  Artificial

<220>
<223>  Synthetic peptide

<400>  67

Met Trp Gln Pro Gln Trp Thr
1               5

<210>  68
<211>  7
<212>  PRT
<213>  Artificial

<220>
<223>  Synthetic peptide

<400>  68

Met Phe Gln Pro Pro Arg Asn
1               5


<210>  69
<211>  7
<212>  PRT
<213>  Artificial

<220>
<223>  Synthetic peptide

<400>  69

Met Phe Gln Pro Arg Phe Pro
1               5


<210>  70
<211>  7
<212>  PRT
<213>  Artificial

<220>
<223>  Synthetic peptide

<400>  70

Met Phe Gln Pro Val Glu Leu
1               5


<210>  71
<211>  7
<212>  PRT
<213>  Artificial

<220>
<223>  Synthetic peptide

<400>  71

Met Tyr Gln Pro Asn Leu Met
1               5


<210>  72
<211>  12
<212>  PRT
<213>  Artificial

<220>
<223>  Synthetic peptide

<400>  72

Met Tyr Gln Pro Asp Pro Pro Pro Thr Lys Asn Pro

1               5                   10


<210>  73
<211>  12
<212>  PRT
<213>  Artificial

<220>
<223>  Synthetic peptide

<400>  73

Met Tyr Gln Pro Asp Pro Pro Pro Thr Lys Asn Val
1               5                   10


<210>  74
<211>  12
<212>  PRT
<213>  Artificial

<220>
<223>  Synthetic peptide

<400>  74

Met Tyr Gln Pro Asp Pro Pro Thr Thr Lys Asn Pro
1               5                   10


<210>  75
<211>  12
<212>  PRT
<213>  Artificial

<220>
<223>  Synthetic peptide

<400>  75

Met Tyr Gln Pro Asp Pro Pro Thr Thr Lys Asn Val
1               5                   10


<210>  76
<211>  12
<212>  PRT
<213>  Artificial

<220>
<223>  Synthetic peptide

<400>  76

Met Tyr Gln Pro Asp Asn Pro Pro Thr Lys Asn Pro
1               5                   10


<210>  77
<211>  12
<212>  PRT
<213>  Artificial

<220>
<223>  Synthetic peptide

<400>  77

Met Tyr Gln Pro Asp Asn Pro Pro Thr Lys Asn Val
1               5                   10

<210>  78
<211>  12
<212>  PRT
<213>  Artificial

<220>
<223>  Synthetic peptide

<400>  78

Met Tyr Gln Pro Asp Asn Pro Thr Thr Lys Asn Pro
1               5                   10

<210>  79
<211>  12
<212>  PRT
<213>  Artificial

<220>
<223>  Synthetic peptide

<400>  79

Met Tyr Gln Pro Asp Asn Pro Thr Thr Lys Asn Val
1               5                   10

<210>  80
<211>  12
<212>  PRT
<213>  Artificial

<220>
<223>  Synthetic peptide

<400>  80

Met Phe Gln Pro Asp Pro Pro Pro Thr Lys Asn Pro
1               5                   10

<210>  81
<211>  12
<212>  PRT
<213>  Artificial

<220>
<223>  Synthetic peptide

<400>  81

Met Phe Gln Pro Asp Pro Pro Pro Thr Lys Asn Val
1               5                   10

<210>  82
<211>  12
<212>  PRT
<213>  Artificial

<220>
<223>  Synthetic peptide

<400>  82

Met Phe Gln Pro Asp Pro Pro Thr Thr Lys Asn Pro
1               5                   10


<210>  83
<211>  12
<212>  PRT
<213>  Artificial

<220>
<223>  Synthetic peptide

<400>  83

Met Phe Gln Pro Asp Pro Pro Thr Thr Lys Asn Val
1               5                   10


<210>  84
<211>  12
<212>  PRT
<213>  Artificial

<220>
<223>  Synthetic peptide

<400>  84

Met Phe Gln Pro Asp Asn Pro Pro Thr Lys Asn Pro
1               5                   10


<210>  85
<211>  12
<212>  PRT
<213>  Artificial

<220>
<223>  Synthetic peptide

<400>  85

Met Phe Gln Pro Asp Asn Pro Pro Thr Lys Asn Val
1               5                   10


<210>  86
<211>  12
<212>  PRT
<213>  Artificial

<220>
<223>  Synthetic peptide

<400>  86

Met Phe Gln Pro Asp Asn Pro Thr Thr Lys Asn Pro
1               5                   10


<210>  87
<211>  12
<212>  PRT
<213>  Artificial

<220>
<223>  Synthetic peptide

<400>  87

Met Phe Gln Pro Asp Asn Pro Thr Thr Lys Asn Val
1               5                   10


<210>  88
<211>  12
<212>  PRT
<213>  Artificial

<220>
<223>  Synthetic peptide

<400>  88

Ser Pro Pro Thr Pro Pro Arg Phe Ser Asp Asp Trp
1               5                   10


<210>  89
<211>  12
<212>  PRT
<213>  Artificial

<220>
<223>  Synthetic peptide

<400>  89

Leu Pro Thr Leu His Thr Ala His Pro Pro Val Gln
1               5                   10


<210>  90
<211>  12
<212>  PRT
<213>  Artificial

<220>
<223>  Synthetic peptide

<400>  90

Gly Thr Gln Thr Arg Ser Ser Tyr Val Leu Thr Ser
1               5                   10


<210>  91
<211>  12
<212>  PRT
<213>  Artificial

<220>
<223>  Synthetic peptide

<400>  91

Asn Pro His Pro Met Trp Asn Pro Thr Ser Tyr Leu
1               5                   10


<210>  92
<211>  12
<212>  PRT

<213>  Artificial

<220>
<223>  Synthetic peptide

<400>  92

Ala Tyr Arg Ala Val Thr Leu Asn Gln Pro Leu Thr
1               5                   10


<210>  93
<211>  12
<212>  PRT
<213>  Artificial

<220>
<223>  Synthetic peptide

<400>  93

Gln Tyr Leu Pro Asp His Arg Ser Ser Gln Pro Asn
1               5                   10


<210>  94
<211>  12
<212>  PRT
<213>  Artificial

<220>
<223>  Synthetic peptide

<400>  94

Leu Pro His Arg Thr Asp Tyr Leu Tyr Thr Pro Glu
1               5                   10


<210>  95
<211>  12
<212>  PRT
<213>  Artificial

<220>
<223>  Synthetic peptide

<400>  95

His Pro Ala Val Gln Pro Pro Leu Ile Tyr Met Phe
1               5                   10


<210>  96
<211>  12
<212>  PRT
<213>  Artificial

<220>
<223>  Synthetic peptide

<400>  96

Val Pro Thr Tyr Glu Ile Ile Ala Arg Gly Ile Arg
1               5                   10


<210>  97

<211>  12
<212>  PRT
<213>  Artificial

<220>
<223>  Synthetic peptide

<400>  97

Ser Pro Ser Asp Met Phe Ile Ser Thr Gln Arg Leu
1               5                   10


<210>  98
<211>  12
<212>  PRT
<213>  Artificial

<220>
<223>  Synthetic peptide

<400>  98

Leu Leu Thr Gln Thr Thr Ala Tyr Ala Pro Met Ser
1               5                   10


<210>  99
<211>  12
<212>  PRT
<213>  Artificial

<220>
<223>  Synthetic peptide

<400>  99

Met Met Asn Ser Asp Pro Lys Ser Phe Leu Ser Leu
1               5                   10


<210>  100
<211>  12
<212>  PRT
<213>  Artificial

<220>
<223>  Synthetic peptide

<400>  100

Met Arg Asn Asp Val Pro Trp Ile Pro Trp Pro Val
1               5                   10


<210>  101
<211>  12
<212>  PRT
<213>  Artificial

<220>
<223>  Synthetic peptide

<400>  101

Thr Leu Pro Tyr Leu Lys Met His Arg Asn Leu Leu
1               5                   10

<210>  102
<211>  7
<212>  PRT
<213>  Artificial

<220>
<223>  Synthetic peptide

<400>  102

Gln Ser Tyr Ser Pro Arg Phe
1               5


<210>  103
<211>  7
<212>  PRT
<213>  Artificial

<220>
<223>  Synthetic peptide

<400>  103

Leu Lys Gln Arg Thr Leu Met
1               5


<210>  104
<211>  7
<212>  PRT
<213>  Artificial

<220>
<223>  Synthetic peptide

<400>  104

Gln Ser Tyr Pro Gln Ala Leu
1               5


<210>  105
<211>  7
<212>  PRT
<213>  Artificial

<220>
<223>  Synthetic peptide

<400>  105

Gln Ser Tyr Ser Pro Lys Tyr
1               5


<210>  106
<211>  7
<212>  PRT
<213>  Artificial

<220>
<223>  Synthetic peptide

<400>  106

Ala Met Tyr Gln Pro Thr His

1                5


<210>  107
<211>  7
<212>  PRT
<213>  Artificial

<220>
<223>  Synthetic peptide

<400>  107

His Trp Tyr His Asn Thr Asp
1               5


<210>  108
<211>  7
<212>  PRT
<213>  Artificial

<220>
<223>  Synthetic peptide

<400>  108

Leu Ser Tyr Ser Pro Arg Tyr
1               5


<210>  109
<211>  7
<212>  PRT
<213>  Artificial

<220>
<223>  Synthetic peptide

<400>  109

Glu Ser Tyr Ser Pro Arg His
1               5


<210>  110
<211>  7
<212>  PRT
<213>  Artificial

<220>
<223>  Synthetic peptide

<400>  110

Gln Ser Tyr Pro Ser Val Tyr
1               5


<210>  111
<211>  7
<212>  PRT
<213>  Artificial

<220>
<223>  Synthetic peptide

<400>  111

Asp Ser Tyr Ser Pro Lys Phe
1               5


<210>  112
<211>  7
<212>  PRT
<213>  Artificial

<220>
<223>  Synthetic peptide

<400>  112

Gln Ser Tyr Pro Ala Lys Phe
1               5


<210>  113
<211>  7
<212>  PRT
<213>  Artificial

<220>
<223>  Synthetic peptide

<400>  113

Arg Ser Ala Ser Ala Leu Trp
1               5


<210>  114
<211>  7
<212>  PRT
<213>  Artificial

<220>
<223>  Synthetic peptide

<400>  114

Glu Ser Tyr Ser Pro Arg Leu
1               5


<210>  115
<211>  7
<212>  PRT
<213>  Artificial

<220>
<223>  Synthetic peptide

<400>  115

Pro Ile Phe Thr Lys Leu His
1               5


<210>  116
<211>  7
<212>  PRT
<213>  Artificial

<220>
<223>  Synthetic peptide

<400>  116

Gln Ser Tyr Pro Tyr Arg Gly
1               5


<210>  117
<211>  7
<212>  PRT
<213>  Artificial

<220>
<223>  Synthetic peptide

<400>  117

Trp Gln Ser Tyr Pro Asn Pro
1               5


<210>  118
<211>  7
<212>  PRT
<213>  Artificial

<220>
<223>  Synthetic peptide

<400>  118

Thr Asn Leu Arg Leu Pro Ala
1               5


<210>  119
<211>  7
<212>  PRT
<213>  Artificial

<220>
<223>  Synthetic peptide

<400>  119

Tyr Gln Ser Tyr Pro Pro Pro
1               5


<210>  120
<211>  7
<212>  PRT
<213>  Artificial

<220>
<223>  Synthetic peptide

<400>  120

Tyr Gln Ser Tyr Pro Met Arg
1               5


<210>  121
<211>  7
<212>  PRT
<213>  Artificial

<220>
<223>  Synthetic peptide

<400>  121

Met Tyr Pro Asp Trp Gln Gln
1               5


<210>  122
<211>  7
<212>  PRT
<213>  Artificial

<220>
<223>  Synthetic peptide

<400>  122

Leu Glu Arg Asp Pro Pro Val
1               5


<210>  123
<211>  7
<212>  PRT
<213>  Artificial

<220>
<223>  Synthetic peptide

<400>  123

Tyr Gln Ser Tyr Pro Asn Trp
1               5


<210>  124
<211>  7
<212>  PRT
<213>  Artificial

<220>
<223>  Synthetic peptide

<400>  124

Leu Val Pro Pro Asp Gly Tyr
1               5


<210>  125
<211>  7
<212>  PRT
<213>  Artificial

<220>
<223>  Synthetic peptide

<400>  125

Ala Pro Phe Trp Gln Trp Met
1               5


<210>  126
<211>  7
<212>  PRT

<213>  Artificial

<220>
<223>  Synthetic peptide

<400>  126

Phe Ser Pro Pro Asn Trp Tyr
1               5


<210>  127
<211>  8
<212>  PRT
<213>  Artificial

<220>
<223>  Synthetic peptide

<400>  127

Phe Ala Pro Trp Tyr Gln Gln Arg
1               5


<210>  128
<211>  7
<212>  PRT
<213>  Artificial

<220>
<223>  Synthetic peptide

<400>  128

Gln Ser Tyr Pro Asn Thr Phe
1               5


<210>  129
<211>  7
<212>  PRT
<213>  Artificial

<220>
<223>  Synthetic peptide

<400>  129

Asp Trp Leu Phe Lys Met Ser
1               5


<210>  130
<211>  6
<212>  PRT
<213>  Artificial

<220>
<223>  Synthetic peptide

<400>  130

Gly Asp Trp Leu His Trp
1               5


<210>  131

<211>  7
<212>  PRT
<213>  Artificial

<220>
<223>  Synthetic peptide

<400>  131

Ser Asp Gly Asp Tyr Gly Ala
1               5


<210>  132
<211>  7
<212>  PRT
<213>  Artificial

<220>
<223>  Synthetic peptide

<400>  132

Gly Pro Ser Trp Gln Ser Thr
1               5


<210>  133
<211>  8
<212>  PRT
<213>  Artificial

<220>
<223>  Synthetic peptide

<400>  133

Val Pro Tyr Pro Asn Gln Met Cys
1               5


<210>  134
<211>  6
<212>  PRT
<213>  Artificial

<220>
<223>  Synthetic peptide

<400>  134

Pro Ser Pro Gly Gln Ile
1               5


<210>  135
<211>  12
<212>  PRT
<213>  Artificial

<220>
<223>  Synthetic peptide

<400>  135

Phe Glu Pro Pro Asn Trp Asp Ser Gly Pro Arg Pro
1               5                   10

<210>  136
<211>  12
<212>  PRT
<213>  Artificial

<220>
<223>  Synthetic peptide

<400>  136

Gly Ile Ala Glu Asp Asp Tyr Gly Ala Leu Ser Trp
1               5                   10


<210>  137
<211>  12
<212>  PRT
<213>  Artificial

<220>
<223>  Synthetic peptide

<400>  137

Tyr Asn Gln Tyr Gln Ser Tyr Pro Pro Ser Leu Gln
1               5                   10


<210>  138
<211>  12
<212>  PRT
<213>  Artificial

<220>
<223>  Synthetic peptide

<400>  138

Tyr Gln Ser Tyr Pro Ser Arg Pro Pro Val Arg Leu
1               5                   10


<210>  139
<211>  12
<212>  PRT
<213>  Artificial

<220>
<223>  Synthetic peptide

<400>  139

Ala Leu Ala Pro Glu Glu Asp Glu Val Tyr Tyr Val
1               5                   10


<210>  140
<211>  12
<212>  PRT
<213>  Artificial

<220>
<223>  Synthetic peptide

<400>  140

Val Leu Leu Pro Glu Ser Asp Glu Ser His Arg Ala

1                   5                        10


<210>  141
<211>  7
<212>  PRT
<213>  Artificial

<220>
<223>  Synthetic peptide

<400>  141

Ile Pro Asn Trp Gln Ser Cys
1               5


<210>  142
<211>  15
<212>  PRT
<213>  Artificial

<220>
<223>  Synthetic peptide

<400>  142

Ser Gly Val Ala Phe Cys Pro Pro Trp Trp Cys Asp Gly Pro Leu
1               5                   10                  15


<210>  143
<211>  15
<212>  PRT
<213>  Artificial

<220>
<223>  Synthetic peptide

<400>  143

Trp Pro Arg His Val Pro Leu Phe Gly Leu Asp Gly Tyr Val Thr
1               5                   10                  15


<210>  144
<211>  15
<212>  PRT
<213>  Artificial

<220>
<223>  Synthetic peptide

<400>  144

Ala Arg Glu Tyr Gly Thr Arg Phe Ser Leu Ile Gly Gly Tyr Arg
1               5                   10                  15


<210>  145
<211>  15
<212>  PRT
<213>  Artificial

<220>
<223>  Synthetic peptide

<400>  145

Ala Ser Arg Arg Leu Leu Pro Ser Asp Val Arg Leu Pro Ser Ser
1               5                   10                  15


<210>  146
<211>  15
<212>  PRT
<213>  Artificial

<220>
<223>  Synthetic peptide

<400>  146

Leu Trp Asp Pro Pro Pro Phe Gly Leu Ser Arg Ile Phe Phe Gly
1               5                   10                  15


<210>  147
<211>  15
<212>  PRT
<213>  Artificial

<220>
<223>  Synthetic peptide

<400>  147

Asp Ala Arg Pro Leu Ala Trp Tyr Glu Glu Pro Ser Phe Trp Met
1               5                   10                  15


<210>  148
<211>  15
<212>  PRT
<213>  Artificial

<220>
<223>  Synthetic peptide

<400>  148

Ser Gly Tyr Ala Arg Pro Phe Tyr Gln Ser Tyr Pro Ala Ala Ser
1               5                   10                  15


<210>  149
<211>  12
<212>  PRT
<213>  Artificial

<220>
<223>  Synthetic peptide

<400>  149

Val Asn Ser Thr Lys Trp Pro Gly Met Pro Ser Phe
1               5                   10


<210>  150
<211>  12
<212>  PRT
<213>  Artificial

<220>
<223>  Synthetic peptide

<400>  150

Val Ser Pro Pro Glu Trp Tyr Pro Leu Ala Ala Asp
1               5                   10


<210>  151
<211>  12
<212>  PRT
<213>  Artificial

<220>
<223>  Synthetic peptide

<400>  151

Thr Gly Pro Lys Leu His Cys Pro Pro Ala Val Cys
1               5                   10


<210>  152
<211>  12
<212>  PRT
<213>  Artificial

<220>
<223>  Synthetic peptide

<400>  152

Phe Lys Asn Pro Ser Gln Ser Tyr Pro Pro Glu Pro
1               5                   10


<210>  153
<211>  12
<212>  PRT
<213>  Artificial

<220>
<223>  Synthetic peptide

<400>  153

Phe Thr Met Glu Arg Asp Pro Pro Ile Ala Arg Val
1               5                   10


<210>  154
<211>  7
<212>  PRT
<213>  Artificial

<220>
<223>  Synthetic peptide

<400>  154

Gly Leu Phe Lys Ile Gln Pro
1               5


<210>  155
<211>  7
<212>  PRT
<213>  Artificial

<220>
<223>  Synthetic peptide

<400>  155

Leu Gly His His Asn Pro His
1               5


<210>  156
<211>  7
<212>  PRT
<213>  Artificial

<220>
<223>  Synthetic peptide

<400>  156

Asn Leu Leu Pro Ala Lys Leu
1               5


<210>  157
<211>  7
<212>  PRT
<213>  Artificial

<220>
<223>  Synthetic peptide

<400>  157

His Thr Ala Leu Ser Thr Ser
1               5


<210>  158
<211>  7
<212>  PRT
<213>  Artificial

<220>
<223>  Synthetic peptide

<400>  158

Thr Pro Gly Ser Gly Gln Phe
1               5


<210>  159
<211>  12
<212>  PRT
<213>  Artificial

<220>
<223>  Synthetic peptide

<400>  159

Phe Ser Pro Asn Asn Ser Glu Thr Thr Asn Tyr Thr
1               5                   10


<210>  160
<211>  12
<212>  PRT

<213>  Artificial

<220>
<223>  Synthetic peptide

<400>  160

Thr Thr Val Pro Asp Thr Trp Val Leu Phe Pro Arg
1               5                   10


<210>  161
<211>  12
<212>  PRT
<213>  Artificial

<220>
<223>  Synthetic peptide

<400>  161

Ser Asn Val Pro Leu His Trp Ile Leu Ser Pro Arg
1               5                   10


<210>  162
<211>  12
<212>  PRT
<213>  Artificial

<220>
<223>  Synthetic peptide

<400>  162

Trp His Phe Glu Trp Trp Arg Ala Thr Pro Ser Gly
1               5                   10


<210>  163
<211>  12
<212>  PRT
<213>  Artificial

<220>
<223>  Synthetic peptide

<400>  163

Asn Tyr Pro Ala Val Val Ser Asn Ser Leu Leu Ala
1               5                   10


<210>  164
<211>  12
<212>  PRT
<213>  Artificial

<220>
<223>  Synthetic peptide

<400>  164

Asp Val Tyr Asp Thr Arg Pro Tyr His Gly Phe Met
1               5                   10


<210>  165

<211>  12
<212>  PRT
<213>  Artificial

<220>
<223>  Synthetic peptide

<400>  165

His Gln Asn Pro Trp Leu Leu Trp Pro Arg Gly Phe
1               5                   10


<210>  166
<211>  15
<212>  PRT
<213>  Artificial

<220>
<223>  Synthetic peptide

<400>  166

Val Ile Asp Trp Leu Leu Cys Pro Arg Gly Phe Ser Ala Cys Met
1               5                   10                  15


<210>  167
<211>  15
<212>  PRT
<213>  Artificial

<220>
<223>  Synthetic peptide

<400>  167

Asp Arg Arg Ile Phe Trp Trp Ser Leu Arg Ser Ala Pro Gly Ala
1               5                   10                  15


<210>  168
<211>  15
<212>  PRT
<213>  Artificial

<220>
<223>  Synthetic peptide

<400>  168

Leu His Ala Trp His Arg Asn Thr Ile Gly Trp Trp Ile Gly Phe
1               5                   10                  15


<210>  169
<211>  7
<212>  PRT
<213>  Artificial

<220>
<223>  Synthetic peptide

<400>  169

Trp Gly Leu Trp Phe Ser Asn
1               5

<210>  170
<211>  7
<212>  PRT
<213>  Artificial

<220>
<223>  Synthetic peptide

<400>  170

Trp Gly Ile Trp Trp Asn Asn
1               5


<210>  171
<211>  7
<212>  PRT
<213>  Artificial

<220>
<223>  Synthetic peptide

<400>  171

Trp Gly Leu Trp Ser Thr Pro
1               5


<210>  172
<211>  7
<212>  PRT
<213>  Artificial

<220>
<223>  Synthetic peptide

<400>  172

Trp Val His Leu Gln Ser Pro
1               5


<210>  173
<211>  7
<212>  PRT
<213>  Artificial

<220>
<223>  Synthetic peptide

<400>  173

Trp Gly Leu Trp Trp His Thr
1               5


<210>  174
<211>  7
<212>  PRT
<213>  Artificial

<220>
<223>  Synthetic peptide

<400>  174

Trp Gly Leu Trp Trp Thr Gly

1               5


<210>  175
<211>  7
<212>  PRT
<213>  Artificial

<220>
<223>  Synthetic peptide

<400>  175

Leu Leu Gly Leu Thr Glu Thr
1               5


<210>  176
<211>  7
<212>  PRT
<213>  Artificial

<220>
<223>  Synthetic peptide

<400>  176

Asp Ser Arg Trp Leu Leu Pro
1               5


<210>  177
<211>  12
<212>  PRT
<213>  Artificial

<220>
<223>  Synthetic peptide

<400>  177

Phe His Trp Trp Pro Arg Thr Gln Asp Pro His Arg
1               5                   10


<210>  178
<211>  12
<212>  PRT
<213>  Artificial

<220>
<223>  Synthetic peptide

<400>  178

Phe His Trp Ser Trp Tyr Thr Pro Ser Arg Pro Ser
1               5                   10


<210>  179
<211>  12
<212>  PRT
<213>  Artificial

<220>
<223>  Synthetic peptide

<400>  179

His Ser Ser Trp Trp Leu Ala Leu Ala Lys Pro Thr
1               5                   10

<210>  180
<211>  12
<212>  PRT
<213>  Artificial

<220>
<223>  Synthetic peptide

<400>  180

Trp His Trp Tyr Pro Arg Phe Ser Pro Pro Ser His
1               5                   10

<210>  181
<211>  12
<212>  PRT
<213>  Artificial

<220>
<223>  Synthetic peptide

<400>  181

Asn Asp Tyr Ser Asp Ser Asn Gln Val Pro Ala Ser
1               5                   10

<210>  182
<211>  12
<212>  PRT
<213>  Artificial

<220>
<223>  Synthetic peptide

<400>  182

Phe His Trp Pro Arg Ser Trp Val Thr Trp Gln Ser
1               5                   10

<210>  183
<211>  12
<212>  PRT
<213>  Artificial

<220>
<223>  Synthetic peptide

<400>  183

His Leu Ser Ser Arg His Leu Phe Val Pro Leu Ser
1               5                   10

<210>  184
<211>  12
<212>  PRT
<213>  Artificial

<220>
<223>  Synthetic peptide

<400>  184

Thr Ile Thr Asp Cys Tyr Pro Ile Cys Val Ser Pro
1               5                   10


<210>  185
<211>  12
<212>  PRT
<213>  Artificial

<220>
<223>  Synthetic peptide

<400>  185

Trp Pro Cys His Pro Ile Cys Leu Ser Pro Arg Gly
1               5                   10


<210>  186
<211>  12
<212>  PRT
<213>  Artificial

<220>
<223>  Synthetic peptide

<400>  186

Ala Met Asp Cys Phe Pro Ile Cys Leu Trp Asn Pro
1               5                   10


<210>  187
<211>  12
<212>  PRT
<213>  Artificial

<220>
<223>  Synthetic peptide

<400>  187

Trp Asn Cys Phe Pro Ile Cys His Ala Ser Gly Leu
1               5                   10


<210>  188
<211>  12
<212>  PRT
<213>  Artificial

<220>
<223>  Synthetic peptide

<400>  188

Val Tyr Ala Leu Gly Cys Trp Pro Ile Cys His Lys
1               5                   10


<210>  189
<211>  12
<212>  PRT
<213>  Artificial

<220>
<223>  Synthetic peptide

<400>  189

His His Val Phe Thr Pro Asn Cys Tyr Pro Ile Cys
1               5                   10


<210>  190
<211>  12
<212>  PRT
<213>  Artificial

<220>
<223>  Synthetic peptide

<400>  190

Trp His Trp Ser Ser Leu Ser Trp Pro Ala Leu Pro
1               5                   10


<210>  191
<211>  15
<212>  PRT
<213>  Artificial

<220>
<223>  Synthetic peptide

<400>  191

Gly Asn Trp Leu Phe Asn Ser Cys Tyr Pro Leu Cys Phe Pro Leu
1               5                   10                  15


<210>  192
<211>  15
<212>  PRT
<213>  Artificial

<220>
<223>  Synthetic peptide

<400>  192

Ala Phe Leu Gly His Ser His Trp Phe Pro Ser Val Ala Ser Arg
1               5                   10                  15


<210>  193
<211>  15
<212>  PRT
<213>  Artificial

<220>
<223>  Synthetic peptide

<400>  193

Gln Leu Leu Cys Phe Pro Ile Cys Arg Pro Glu Pro Pro Val Ser
1               5                   10                  15


<210>  194
<211>  7
<212>  PRT

<213>  Artificial

<220>
<223>  Synthetic peptide

<400>  194

Trp Tyr Thr Lys Ala Pro Tyr
1               5


<210>  195
<211>  7
<212>  PRT
<213>  Artificial

<220>
<223>  Synthetic peptide

<400>  195

Trp Tyr His Lys Ala Pro Tyr
1               5


<210>  196
<211>  7
<212>  PRT
<213>  Artificial

<220>
<223>  Synthetic peptide

<400>  196

Tyr Tyr Gln Arg Ala Pro Tyr
1               5


<210>  197
<211>  12
<212>  PRT
<213>  Artificial

<220>
<223>  Synthetic peptide

<400>  197

His Trp Lys His Pro Trp Gly Ala Trp Asp Thr Leu
1               5                   10


<210>  198
<211>  12
<212>  PRT
<213>  Artificial

<220>
<223>  Synthetic peptide

<400>  198

Trp His Trp Gln Trp Thr Pro Trp Ser Ile Gln Pro
1               5                   10


<210>  199

<211>  12
<212>  PRT
<213>  Artificial

<220>
<223>  Synthetic peptide

<400>  199

Trp His Lys Asn Trp Trp Pro Pro Ser Thr Pro Asn
1               5                   10


<210>  200
<211>  12
<212>  PRT
<213>  Artificial

<220>
<223>  Synthetic peptide

<400>  200

Ser Met Ser Ser Met Leu Leu Ala Ala Gln Thr Val
1               5                   10


<210>  201
<211>  12
<212>  PRT
<213>  Artificial

<220>
<223>  Synthetic peptide

<400>  201

Gln Ser His Tyr Arg His Ile Ser Pro Ala Gln Val
1               5                   10


<210>  202
<211>  12
<212>  PRT
<213>  Artificial

<220>
<223>  Synthetic peptide

<400>  202

Ser Met Ser Gln Pro Lys Ser Gln Val Asn Ala His
1               5                   10


<210>  203
<211>  12
<212>  PRT
<213>  Artificial

<220>
<223>  Synthetic peptide

<400>  203

Trp His Trp Thr Phe Tyr Thr Pro Leu Glu Ser Thr
1               5                   10

<210>  204
<211>  12
<212>  PRT
<213>  Artificial

<220>
<223>  Synthetic peptide

<400>  204

Glu Ser Leu Ser Thr Asp Thr Tyr Ala Ile Leu Leu
1               5                   10


<210>  205
<211>  12
<212>  PRT
<213>  Artificial

<220>
<223>  Synthetic peptide

<400>  205

His Ser Ser Trp Tyr Ile Gln His Phe Pro Pro Leu
1               5                   10


<210>  206
<211>  12
<212>  PRT
<213>  Artificial

<220>
<223>  Synthetic peptide

<400>  206

Ser Thr Tyr Leu Asn Gly Pro Thr Gly Val Asp Leu
1               5                   10


<210>  207
<211>  12
<212>  PRT
<213>  Artificial

<220>
<223>  Synthetic peptide

<400>  207

Gln His Lys Thr Ser Ile Thr Gly His Leu Glu Pro
1               5                   10


<210>  208
<211>  12
<212>  PRT
<213>  Artificial

<220>
<223>  Synthetic peptide

<400>  208

Phe His Arg Trp Pro Thr Trp Pro Leu Pro Ser Pro

1               5                   10


<210>  209
<211>  12
<212>  PRT
<213>  Artificial

<220>
<223>  Synthetic peptide

<400>  209

Val Pro Pro Trp Val Ser Val Arg Thr Gly Pro Gly
1               5                   10


<210>  210
<211>  12
<212>  PRT
<213>  Artificial

<220>
<223>  Synthetic peptide

<400>  210

Thr Leu Val Tyr Gln Pro Pro Trp Tyr Arg Ile Ala
1               5                   10


<210>  211
<211>  12
<212>  PRT
<213>  Artificial

<220>
<223>  Synthetic peptide

<400>  211

Phe His Gln Arg Leu Trp Trp Pro Thr His Thr Pro
1               5                   10


<210>  212
<211>  11
<212>  PRT
<213>  Artificial

<220>
<223>  Synthetic peptide

<400>  212

Trp His Trp Arg Leu Tyr Ser Ala Asn Thr Pro
1               5                   10


<210>  213
<211>  15
<212>  PRT
<213>  Artificial

<220>
<223>  Synthetic peptide

<400>  213

His Ala Ala Phe Glu Pro Arg Gly Asp Val Arg His Thr Leu Leu
1               5                   10                  15


<210>  214
<211>  15
<212>  PRT
<213>  Artificial

<220>
<223>  Synthetic peptide

<400>  214

Ser Ile Trp Asp Leu Pro Leu Gln Tyr Arg Gly Phe Gly Thr Ser
1               5                   10                  15


<210>  215
<211>  15
<212>  PRT
<213>  Artificial

<220>
<223>  Synthetic peptide

<400>  215

Leu Trp Arg Leu Arg Gly Gly Ser Phe Pro Val Ile Ser His Gly
1               5                   10                  15


<210>  216
<211>  15
<212>  PRT
<213>  Artificial

<220>
<223>  Synthetic peptide

<400>  216

Arg Asn Ala Leu His Ser Leu Arg Thr Leu Ser Ser Ser Trp Val
1               5                   10                  15


<210>  217
<211>  12
<212>  PRT
<213>  Artificial

<220>
<223>  Synthetic peptide

<400>  217

Phe His Arg His Trp Trp Pro Pro Thr Leu Ser Thr
1               5                   10


<210>  218
<211>  12
<212>  PRT
<213>  Artificial

<220>
<223>  Synthetic peptide

<400>  218

Arg Pro His Leu Leu Asp Trp Glu Leu Asn Pro Val
1               5                   10


<210>  219
<211>  12
<212>  PRT
<213>  Artificial

<220>
<223>  Synthetic peptide

<400>  219

Phe His Trp Arg Trp Ser Thr Phe Pro Glu Tyr Pro
1               5                   10


<210>  220
<211>  12
<212>  PRT
<213>  Artificial

<220>
<223>  Synthetic peptide

<400>  220

Val Trp Ala Val Ser Leu Pro Trp Tyr Arg Tyr Pro
1               5                   10


<210>  221
<211>  12
<212>  PRT
<213>  Artificial

<220>
<223>  Synthetic peptide

<400>  221

Leu Asp Thr Tyr Trp Tyr Arg Glu His Phe Arg Arg
1               5                   10


<210>  222
<211>  12
<212>  PRT
<213>  Artificial

<220>
<223>  Synthetic peptide

<400>  222

Val His Trp Arg Trp Trp Asp Gln Arg Val Pro Met
1               5                   10


<210>  223
<211>  7
<212>  PRT
<213>  Artificial

<220>
<223>  Synthetic peptide

<400>  223

Leu Pro Trp Tyr Gln Leu Thr
1               5


<210>  224
<211>  7
<212>  PRT
<213>  Artificial

<220>
<223>  Synthetic peptide

<400>  224

Val Pro Trp Phe Arg Ala Pro
1               5


<210>  225
<211>  7
<212>  PRT
<213>  Artificial

<220>
<223>  Synthetic peptide

<400>  225

Trp His Pro Pro Gln Pro Ser
1               5


<210>  226
<211>  7
<212>  PRT
<213>  Artificial

<220>
<223>  Synthetic peptide

<400>  226

Phe His Glu Asn Trp Pro Ser
1               5


<210>  227
<211>  7
<212>  PRT
<213>  Artificial

<220>
<223>  Synthetic peptide

<400>  227

Phe Trp Trp Gln Val Pro Ala
1               5


<210>  228
<211>  7
<212>  PRT

<213>  Artificial

<220>
<223>  Synthetic peptide

<400>  228

Thr Gln Trp Tyr Gln Ile Ala
1               5


<210>  229
<211>  7
<212>  PRT
<213>  Artificial

<220>
<223>  Synthetic peptide

<400>  229

Leu Pro Trp Phe Gln Leu Pro
1               5


<210>  230
<211>  7
<212>  PRT
<213>  Artificial

<220>
<223>  Synthetic peptide

<400>  230

Thr Pro Leu Ser Lys Ser Thr
1               5


<210>  231
<211>  7
<212>  PRT
<213>  Artificial

<220>
<223>  Synthetic peptide

<400>  231

Leu Pro Trp Tyr Ala Thr Pro
1               5


<210>  232
<211>  6
<212>  PRT
<213>  Artificial

<220>
<223>  Synthetic peptide

<400>  232

Leu Pro Trp Tyr Arg His
1               5


<210>  233

<211>  7
<212>  PRT
<213>  Artificial

<220>
<223>  Synthetic peptide

<400>  233

Ile Pro Trp Tyr Lys Ile Thr
1               5


<210>  234
<211>  12
<212>  PRT
<213>  Artificial

<220>
<223>  Synthetic peptide

<400>  234

Phe His Ser Thr Trp Pro Trp Arg Glu Ala Glu Gly
1               5                   10


<210>  235
<211>  12
<212>  PRT
<213>  Artificial

<220>
<223>  Synthetic peptide

<400>  235

Phe His Ala Asn Trp Pro Gln Ser Ala Arg Asp Val
1               5                   10


<210>  236
<211>  12
<212>  PRT
<213>  Artificial

<220>
<223>  Synthetic peptide

<400>  236

Phe His Ser Asp Trp Pro Gly Gln Thr Phe Thr Trp
1               5                   10


<210>  237
<211>  12
<212>  PRT
<213>  Artificial

<220>
<223>  Synthetic peptide

<400>  237

Phe His Glu Asn Trp Ser Thr Arg Pro Thr Thr Arg
1               5                   10

<210>  238
<211>  12
<212>  PRT
<213>  Artificial

<220>
<223>  Synthetic peptide

<400>  238

Phe His Ser Val Tyr Pro Trp Arg Glu Ala Glu Gly
1               5                   10


<210>  239
<211>  12
<212>  PRT
<213>  Artificial

<220>
<223>  Synthetic peptide

<400>  239

Phe His Ser Asn Trp Pro Ser Ala Tyr Thr Ala Arg
1               5                   10


<210>  240
<211>  12
<212>  PRT
<213>  Artificial

<220>
<223>  Synthetic peptide

<400>  240

Phe His Ser Asn Trp Pro Ser Leu Ile Arg Ala Arg
1               5                   10


<210>  241
<211>  12
<212>  PRT
<213>  Artificial

<220>
<223>  Synthetic peptide

<400>  241

Ala Gly Tyr Gln Ile Gly Met Pro Asn Pro Leu Leu
1               5                   10


<210>  242
<211>  12
<212>  PRT
<213>  Artificial

<220>
<223>  Synthetic peptide

<400>  242

Phe His Trp Arg Tyr Pro Leu Pro Leu Pro Gly Gln

1                   5                   10


<210>  243
<211>  12
<212>  PRT
<213>  Artificial

<220>
<223>  Synthetic peptide

<400>  243

Ser Pro Thr Ser Phe Arg Gln Val Phe Gly Phe Tyr
1               5                   10


<210>  244
<211>  13
<212>  PRT
<213>  Artificial

<220>
<223>  Synthetic peptide

<400>  244

Ala Leu Pro Glu Leu Ser Ser Leu Pro Glu Ser Ala Arg
1               5                   10


<210>  245
<211>  12
<212>  PRT
<213>  Artificial

<220>
<223>  Synthetic peptide

<400>  245

Leu His Trp Trp Pro Thr Tyr Gly Asn Asn Gly Met
1               5                   10


<210>  246
<211>  12
<212>  PRT
<213>  Artificial

<220>
<223>  Synthetic peptide

<400>  246

Phe His Arg Pro Tyr Tyr Trp Pro Pro Thr Pro Leu
1               5                   10


<210>  247
<211>  12
<212>  PRT
<213>  Artificial

<220>
<223>  Synthetic peptide

<400>  247

Phe His Trp Arg Leu Pro Tyr Pro Leu Pro Ser Ser
1               5                   10

<210>  248
<211>  12
<212>  PRT
<213>  Artificial

<220>
<223>  Synthetic peptide

<400>  248

Ile His Trp Trp Val Lys Ser Pro Pro Pro Gly Ser
1               5                   10

<210>  249
<211>  12
<212>  PRT
<213>  Artificial

<220>
<223>  Synthetic peptide

<400>  249

Ser His Trp Trp Thr Ser Ile Leu Ala Thr Pro Ser
1               5                   10

<210>  250
<211>  12
<212>  PRT
<213>  Artificial

<220>
<223>  Synthetic peptide

<400>  250

Phe His Trp His Leu Gln Pro Gln Leu Trp Ser Tyr
1               5                   10

<210>  251
<211>  12
<212>  PRT
<213>  Artificial

<220>
<223>  Synthetic peptide

<400>  251

Leu His Arg Glu Trp Ile Tyr Pro Tyr Leu Ile Ser
1               5                   10

<210>  252
<211>  12
<212>  PRT
<213>  Artificial

<220>
<223>  Synthetic peptide

<400>  252

Gly Gln Lys Thr His Asn Pro Phe His Leu His Pro
1               5                   10


<210>  253
<211>  12
<212>  PRT
<213>  Artificial

<220>
<223>  Synthetic peptide

<400>  253

Ala Thr Trp Ser His His Leu Ser Ser Ala Gly Leu
1               5                   10


<210>  254
<211>  12
<212>  PRT
<213>  Artificial

<220>
<223>  Synthetic peptide

<400>  254

Phe His Arg His Tyr Tyr Pro Trp Ala Leu Ile Gln
1               5                   10


<210>  255
<211>  12
<212>  PRT
<213>  Artificial

<220>
<223>  Synthetic peptide

<400>  255

His Ser Leu Lys His Thr Gln Met Ser Tyr Ser Ser
1               5                   10


<210>  256
<211>  12
<212>  PRT
<213>  Artificial

<220>
<223>  Synthetic peptide

<400>  256

Ser Val Ser Val Gly Met Lys Pro Ser Pro Arg Pro
1               5                   10


<210>  257
<211>  12
<212>  PRT
<213>  Artificial

<220>
<223>  Synthetic peptide

<400>  257

Trp Pro His Gln Val His Lys His Ile Tyr Arg Gln
1               5                   10


<210>  258
<211>  12
<212>  PRT
<213>  Artificial

<220>
<223>  Synthetic peptide

<400>  258

Ala Pro Pro Tyr Pro Gly Pro Leu Pro Leu Ser Leu
1               5                   10


<210>  259
<211>  13
<212>  PRT
<213>  Artificial

<220>
<223>  Synthetic peptide

<400>  259

Ala Ala Pro Pro Tyr Val Phe Cys Ser Leu Ser Pro Arg
1               5                   10


<210>  260
<211>  15
<212>  PRT
<213>  Artificial

<220>
<223>  Synthetic peptide

<400>  260

Thr Val Asp Leu Ser Pro Ala Phe Leu Phe Phe Gly Arg Leu Ala
1               5                   10                  15


<210>  261
<211>  15
<212>  PRT
<213>  Artificial

<220>
<223>  Synthetic peptide

<400>  261

Ser Leu Ala Pro Tyr Ser Leu Arg Ile Leu Arg Val Gly Ser Ala
1               5                   10                  15


<210>  262
<211>  15
<212>  PRT

<213>  Artificial

<220>
<223>  Synthetic peptide

<400>  262

Arg Asn Val Leu Pro Ile Phe Asn Asp Val Tyr Trp Ile Ala Leu
1               5                   10                  15


<210>  263
<211>  15
<212>  PRT
<213>  Artificial

<220>
<223>  Synthetic peptide

<400>  263

Arg Asn Val Pro Pro Ile Phe Asn Asp Val Tyr Trp Ile Ala Phe
1               5                   10                  15


<210>  264
<211>  7
<212>  PRT
<213>  Artificial

<220>
<223>  Synthetic peptide

<400>  264

Ile Asn Asn Thr Phe Thr Trp
1               5


<210>  265
<211>  7
<212>  PRT
<213>  Artificial

<220>
<223>  Synthetic peptide

<400>  265

Tyr Glu Leu Asn Leu Met Thr
1               5


<210>  266
<211>  12
<212>  PRT
<213>  Artificial

<220>
<223>  Synthetic peptide

<400>  266

Trp Gly Ile Thr Val Glu Thr Ala Tyr Gly Thr Ala
1               5                   10


<210>  267

<211>  12
<212>  PRT
<213>  Artificial

<220>
<223>  Synthetic peptide

<400>  267

Asn Gln Phe Leu Leu Trp Glu Thr Arg Ser Met Arg
1               5                   10


<210>  268
<211>  12
<212>  PRT
<213>  Artificial

<220>
<223>  Synthetic peptide

<400>  268

Asn Val Ser Leu Thr Asn Leu Thr Tyr Lys Pro Arg
1               5                   10


<210>  269
<211>  12
<212>  PRT
<213>  Artificial

<220>
<223>  Synthetic peptide

<400>  269

Tyr Thr Pro Pro Asp Trp Ser Trp Trp Pro Ala Pro
1               5                   10


<210>  270
<211>  12
<212>  PRT
<213>  Artificial

<220>
<223>  Synthetic peptide

<400>  270

Ile Lys Asn Thr Ser Leu Gln Gln Leu Val Thr Ala
1               5                   10


<210>  271
<211>  12
<212>  PRT
<213>  Artificial

<220>
<223>  Synthetic peptide

<400>  271

Glu Trp Leu Ala Tyr Asp Gly Ile Arg Ala Tyr Ser
1               5                   10

<210>  272
<211>  12
<212>  PRT
<213>  Artificial

<220>
<223>  Synthetic peptide

<400>  272

His Ala Lys Val Gln Val Ser Ser Pro Phe Pro Pro
1               5                   10


<210>  273
<211>  7
<212>  PRT
<213>  Artificial

<220>
<223>  Synthetic peptide

<400>  273

Leu Ser Ser Gln Phe Lys Gln
1               5


<210>  274
<211>  7
<212>  PRT
<213>  Artificial

<220>
<223>  Synthetic peptide

<400>  274

Gly Thr Phe Lys Trp Tyr Gln
1               5


<210>  275
<211>  7
<212>  PRT
<213>  Artificial

<220>
<223>  Synthetic peptide

<400>  275

Thr Lys Met Asp Gln Ser Thr
1               5


<210>  276
<211>  12
<212>  PRT
<213>  Artificial

<220>
<223>  Synthetic peptide

<400>  276

Ile Pro Glu Glu Ala Leu Arg Ala Arg Phe Lys Thr

1               5                   10


<210>  277
<211>  12
<212>  PRT
<213>  Artificial

<220>
<223>  Synthetic peptide

<400>  277

Ser Phe Gln Asp Ala Leu Leu Ser Arg Trp Tyr Ser
1               5                   10


<210>  278
<211>  15
<212>  PRT
<213>  Artificial

<220>
<223>  Synthetic peptide

<400>  278

Ser Asn Leu Arg Ser Trp Leu Phe Pro Phe Asp Arg Val Gly Asn
1               5                   10                  15


<210>  279
<211>  15
<212>  PRT
<213>  Artificial

<220>
<223>  Synthetic peptide

<400>  279

Pro Gly Pro Met Phe Gly Gly Ser Tyr Ile Pro Ser Leu His His
1               5                   10                  15


<210>  280
<211>  7
<212>  PRT
<213>  Artificial

<220>
<223>  Synthetic peptide

<400>  280

Tyr Pro Gln Gly Leu Trp Arg
1               5


<210>  281
<211>  7
<212>  PRT
<213>  Artificial

<220>
<223>  Synthetic peptide

<400>  281

Tyr Pro Gln Gly Glu Trp Arg
1               5


<210>  282
<211>  15
<212>  PRT
<213>  Artificial

<220>
<223>  Synthetic peptide

<400>  282

Gly Pro Ser Tyr Tyr Ala Leu Ile Thr Arg Tyr Leu Gly Ala Ala
1               5                   10                  15


<210>  283
<211>  15
<212>  PRT
<213>  Artificial

<220>
<223>  Synthetic peptide

<400>  283

Gly Leu Ser Tyr Tyr Ala Leu Ile Thr Arg Tyr Leu Gly Ala Ala
1               5                   10                  15


<210>  284
<211>  7
<212>  PRT
<213>  Artificial

<220>
<223>  Synthetic peptide

<400>  284

Trp Pro Pro Ile Ser Arg His
1               5


<210>  285
<211>  12
<212>  PRT
<213>  Artificial

<220>
<223>  Synthetic peptide

<400>  285

Ala His Ser Trp Leu Pro Gly Ala Gly Leu Leu Met
1               5                   10


<210>  286
<211>  12
<212>  PRT
<213>  Artificial

<220>
<223>  Synthetic peptide

<400>  286

Ser Leu Thr His Ser Pro Arg Thr Pro Ile Leu Ala
1               5                   10


<210>  287
<211>  12
<212>  PRT
<213>  Artificial

<220>
<223>  Synthetic peptide

<400>  287

Phe Pro Asn Leu Arg Glu Arg His Glu Pro Leu Phe
1               5                   10


<210>  288
<211>  15
<212>  PRT
<213>  Artificial

<220>
<223>  Synthetic peptide

<400>  288

Gly His Ser Tyr His Ala Leu Ile Thr Arg Tyr Leu Gly Ala Ala
1               5                   10                  15


<210>  289
<211>  15
<212>  PRT
<213>  Artificial

<220>
<223>  Synthetic peptide

<400>  289

Trp Ala Ser Tyr Tyr Ala Leu Ile Thr Arg Tyr Leu Gly Ala Ala
1               5                   10                  15


<210>  290
<211>  15
<212>  PRT
<213>  Artificial

<220>
<223>  Synthetic peptide

<400>  290

Asp Phe Ala Gln Ala Leu Phe Leu Arg Tyr Val Val Ser Gly Leu
1               5                   10                  15


<210>  291
<211>  15
<212>  PRT
<213>  Artificial

<220>
<223>  Synthetic peptide

<400>  291

His Arg Trp Met Pro His Val Phe Ala Val Arg Gln Gly Ala Leu
1               5                   10                  15


<210>  292
<211>  15
<212>  PRT
<213>  Artificial

<220>
<223>  Synthetic peptide

<400>  292

Trp Ala Ser Phe Tyr Ala Ser Ser Tyr Arg Asp Ser Arg Leu Leu
1               5                   10                  15


<210>  293
<211>  15
<212>  PRT
<213>  Artificial

<220>
<223>  Synthetic peptide

<400>  293

Arg Leu Pro Ala Thr Ile Arg Ala Leu Leu Gly Arg Asp Val Arg
1               5                   10                  15


<210>  294
<211>  15
<212>  PRT
<213>  Artificial

<220>
<223>  Synthetic peptide

<400>  294

Arg Leu Pro Ala Thr Ile Arg Ala Leu Leu Gly Arg Asp Val Trp
1               5                   10                  15


<210>  295
<211>  7
<212>  PRT
<213>  Artificial

<220>
<223>  Synthetic peptide

<400>  295

Val Pro Arg His Asn Pro Ile
1               5


<210>  296
<211>  15
<212>  PRT

<213>  Artificial

<220>
<223>  Synthetic peptide

<400>  296

Pro Lys Ala Phe Gln Tyr Gly Gly Arg Ala Val Gly Gly Leu Trp
1               5                   10                  15


<210>  297
<211>  15
<212>  PRT
<213>  Artificial

<220>
<223>  Synthetic peptide

<400>  297

Asp Ser Gly Ala Leu Phe Asn His Ile Phe Met Pro Gly Pro Phe
1               5                   10                  15


<210>  298
<211>  15
<212>  PRT
<213>  Artificial

<220>
<223>  Synthetic peptide

<400>  298

Leu His Ser Ser His Leu Pro Pro Asp Asp Arg Arg Trp Gly Leu
1               5                   10                  15


<210>  299
<211>  15
<212>  PRT
<213>  Artificial

<220>
<223>  Synthetic peptide

<400>  299

Pro Arg Gln Phe Pro Leu Arg Asp Leu Tyr Thr Phe Arg Tyr Trp
1               5                   10                  15


<210>  300
<211>  15
<212>  PRT
<213>  Artificial

<220>
<223>  Synthetic peptide

<400>  300

Gly Ala Ala Asn Leu Tyr Val Ser Ser Phe Leu Ile Pro Leu His
1               5                   10                  15


<210>  301

<211>  15
<212>  PRT
<213>  Artificial

<220>
<223>  Synthetic peptide

<400>  301

Arg Asn Val Pro Pro Ile Phe Asn Asp Val Tyr Trp Ile Ala Leu
1               5                   10                  15


<210>  302
<211>  15
<212>  PRT
<213>  Artificial

<220>
<223>  Synthetic peptide

<400>  302

Gln Pro Arg Gly Val Thr Val His Gly Asp Ala Trp Arg Val Ala
1               5                   10                  15


<210>  303
<211>  15
<212>  PRT
<213>  Artificial

<220>
<223>  Synthetic peptide

<400>  303

Gly Leu Asp Leu Leu Gly Asp Val Arg Ile Pro Val Val Arg Arg
1               5                   10                  15


<210>  304
<211>  15
<212>  PRT
<213>  Artificial

<220>
<223>  Synthetic peptide

<400>  304

Ala Tyr Trp Asp Leu Tyr Gly Val Gly Phe Ala Phe Ser Ala Pro
1               5                   10                  15


<210>  305
<211>  15
<212>  PRT
<213>  Artificial

<220>
<223>  Synthetic peptide

<400>  305

Ile Trp Thr Ile Thr Gly Ser Thr Lys Gln Ala Phe Asp Arg Ser
1               5                   10                  15

<210>  306
<211>  15
<212>  PRT
<213>  Artificial

<220>
<223>  Synthetic peptide

<400>  306

Glu Asp Trp Phe Ser Ala Ser Ile Arg Arg Val Pro Thr Phe Ala
1               5                   10                  15


<210>  307
<211>  15
<212>  PRT
<213>  Artificial

<220>
<223>  Synthetic peptide

<400>  307

Ala Val Lys Leu Arg Pro Ser Ser Cys Trp Leu Lys Ser Thr Cys
1               5                   10                  15


<210>  308
<211>  15
<212>  PRT
<213>  Artificial

<220>
<223>  Synthetic peptide

<400>  308

Arg Phe Asp Gly Ile Asp Leu Arg Val Ser Phe Val Ser Arg Pro
1               5                   10                  15


<210>  309
<211>  15
<212>  PRT
<213>  Artificial

<220>
<223>  Synthetic peptide

<400>  309

Ala Ser Leu His Pro Val Pro Lys Thr Trp Phe Ser Leu Leu Ser
1               5                   10                  15


<210>  310
<211>  7
<212>  PRT
<213>  Artificial

<220>
<223>  Synthetic peptide

<400>  310

Leu Ala Pro Pro Pro Ser Pro

1               5


<210>  311
<211>  15
<212>  PRT
<213>  Artificial

<220>
<223>  Synthetic peptide

<400>  311

Ala Val Thr Phe Asn Ser Tyr Phe Gly Phe Ser Thr Thr Ser Val
1               5                   10                  15